# EUROPÄISCHE PATENTSCHRIFT

(11) **EP 4 376 730 B1**
(45) Veröffentlichungstag und Bekanntmachung des Hinweises auf die Patenterteilung: **12.03.2025**
(21) Anmeldenummer: 22757861.4
(22) Anmeldetag: 25.07.2022
(51) Int. Cl.: A61B 17/29, A61B 34/00, A61B 17/00

(54) **CHIRURGISCHES INSTRUMENT UND LENKGETRIEBE DAFÜR**
SURGICAL INSTRUMENT AND STEERING GEAR FOR SAME
INSTRUMENT CHIRURGICAL ET BOÎTIER DE DIRECTION POUR CELUI-CI

(30) Priorität: 28.07.2021 DE 102021119519
(43) Veröffentlichungstag der Anmeldung: 05.06.2024
(73) Patentinhaber: Karl Storz SE & Co. KG, 78532 Tuttlingen (DE)
(72) Erfinder: GRÜNER, Sven Axel, 78532 Tuttlingen (DE); LÄNGLE, Dominik, 78532 Tuttlingen (DE); SCHNEIDER, Janosz, 78532 Tuttlingen (DE); STEFAN, Jochen, 78532 Tuttlingen (DE)
(86) Internationale Anmeldenummer: PCT/EP2022/070808
(87) Internationale Veröffentlichungsnummer: WO 2023/006672

(56) Entgegenhaltungen:
- DE-A1- 102019 121 092
- US-A- 5 454 827
- US-A1- 2010 228 284

## Beschreibung

Die Erfindung betrifft ein Lenkgetriebe eines chirurgischen Instruments zur Abwinkelung einer Werkzeugspitze mittels einer räumlich ausrichtbaren Taumelscheibe, sowie ein chirurgisches Instrument, das ein solches Lenkgetriebe aufweist.

Aus dem Stand der Technik sind chirurgische Instrumente bekannt, die manuell oder von einem Roboter geführt werden können und die Werkzeuge aufweisen, deren Werkzeugspitze mittels mehrerer ineinandergreifender Schwenkglieder verschwenkt werden kann. Diese Schwenkglieder sind mit einer Vielzahl Lenkdrähte oder -seile verbunden, um eine feinfühlige Steuerung der Werkzeugspitze zu erreichen. Mit vielen dünnen Lenkdrähten gegenüber wenigen dickeren Lenkdrähten kann eine gleichmäßigere Kraftverteilung in alle Abwinkelungsrichtungen erzielt werden.

Aus US 5,454,827 B2 ist bekannt, solche Lenkdrähte mit einer proximalseitig in einer Betätigungseinheit angeordneten räumlich verstellbaren Scheibe zu koppeln, die über eine Stange mit einem manuell betätigbaren Steuerhebel verbunden ist, sodass eine Bewegung der räumlich verstellbaren Taumelscheibe eine entsprechende relative Bewegung der distalseitigen Schwenkglieder und somit ein Verschwenken der Werkzeugspitze verursacht.

Die Ausbildung des Antriebs für die Lenkdrähte mit der räumlich verstellbaren Taumelscheibe, an der alle vier Lenkdrähte gelagert sind, hat den Vorteil, dass dies eine räumlich kompakte Bauweise ermöglicht und nur ein Bauteil bewegt werden muss, um alle Lenkdrähte ansprechen zu können. Diese Konstruktion bringt mit sich, dass die Verwendung einer nur geringen Anzahl von Lenkdrähten möglich ist, und dass die als Antrieb für die Lenkdrähte dienenden räumlich verstellbaren Scheibe manuell zu betätigen ist, was beides die Feinfühligkeit und Reproduzierbarkeit der Verstellung der distalseitigen Schwenkglieder beeinflusst.

In der US 7,699,855 B2 ist ein chirurgisches Instrument offenbart, das eine Schnittstelle aufweist, um das Instrument mit einem Roboter-Arm verbinden zu können. Dabei sind alle Antriebe, die das Instrument steuern, in dem Roboter-Arm angeordnet. Die Übertragung der Drehwinkel von Antrieben zum Instrument erfolgt über Kupplungsscheiben in einer gemeinsamen Trenn-Ebene. Auch in der US 2010/228284 A1 sind Mechanismen zum Steuern eines Gelenks in der chirurgischen Robotik beschrieben.

Ferner ist aus DE 10 2019 121 092 A1 bekannt, in einem chirurgischen Instrument mit einem kompakten Lenkgetriebe die Stellwinkel von zwei Antrieben direkt auf die Taumelscheibe zu übertragen, um diese zur Steuerung der Werkzeugspitze auszurichten. Dazu werden an der Taumelscheibe Lenkdrähte befestigt, sodass sich die Werkzeugspitze durch Ausrichtung der Taumelscheibe stufenlos und flüssig steuern lässt. Dazu weist das bekannte Lenkgetriebe zwei um 180 ° zueinander versetzte, auf einer gemeinsamen Drehachse, die senkrecht zu einer Instrumentenlängsachse verläuft, angeordnete Antriebskegelräder mit jeweils einem zugeordneten Motor auf. Die Taumelscheibe ist zwischen den Antriebskegelrädern angeordnet und in einem Lenkring gelagert, der drehfest mit einem dritten Kegelrad verbunden ist, das mit den beiden Antriebskegelrädern in Eingriff steht und um eine Drehachse drehbar ist, die senkrecht zu der Instrumentenlängsachse und senkrecht zu der gemeinsamen Drehachse der Antriebskegelräder verläuft. Die Verzahnungskette wird durch ein viertes Kegelrad ergänzt, das auf der Drehachse des dritten Kegelrads um 180 ° versetzt zum dritten Kegelrad angeordnet ist und mit den beiden Antriebskegelrädern in Eingriff steht, wobei der Lenkring frei drehbar im vierten Kegelrad gelagert ist. Der auf diese Weise geschlossene Verzahnungsring sichert den Eingriff aller Kegelräder miteinander und ermöglicht eine gleichmäßig umlaufende Kraftverteilung.

Ausgehend von diesem Stand der Technik ist es Aufgabe der vorliegenden Erfindung, eine alternative Führung des Lenkrings unter Sicherung des Eingriffs der Antriebskegelräder mit dem Kegelrad des Lenkrings bereitzustellen.

Diese Aufgabe wird durch ein Lenkgetriebe mit den Merkmalen des Anspruchs 1 gelöst. Die weitere Aufgabe der Bereitstellung eines chirurgischen Instruments mit einer alternativen Führung des Lenkrings wird durch das chirurgische Instrument mit den Merkmalen des abhängigen Anspruchs 14 gelöst.

Weiterbildungen es Lenkgetriebes und des chirurgischen Instruments sind in den jeweiligen Unteransprüchen ausgeführt.

Eine erste Ausführungsform des erfindungsgemäßen Lenkgetriebes ist für ein chirurgisches Instrument vorgesehen und ausgebildet ist. Es wird am proximalen Ende eines Schafts angeordnet, der eine Längsachse B definiert und am distalen Ende eine Abwinkelungsmechanik aufweist, die durch eine räumlich ausrichtbare Taumelscheibe steuerbar ist. Das erfindungsgemäße Lenkgetriebe hat ein erstes Antriebsrad und ein zweites Antriebsrad mit jeweils einem zugeordneten Motor, wobei das zweite Antriebsrad um 180 ° zum ersten Antriebsrad versetzt auf einer mit dem ersten Antriebsrad gemeinsamen, senkrecht, d. h. rechtwinklig, zur Längsachse verlaufenden Drehachse A angeordnet ist. Die Taumelscheibe ist dabei zwischen dem ersten Antriebsrad und dem zweiten Antriebsrad in einem Lenkring gelagert, der drehfest mit einem dritten Zahnrad verbunden ist, das mit dem ersten Antriebsrad und dem zweiten Antriebsrad in Eingriff steht und um eine Drehachse C drehbar ist, die senkrecht zu der Längsachse B und der gemeinsamen Drehachse A der Antriebsräder verläuft. Vorteilhaft weist das Lenkgetriebe einen Haltebügel auf, der drehbar um die gemeinsame Drehachse der Antriebsräder gelagert ist, wobei der Lenkring zudem in dem Haltebügel drehbar um die Drehachse des dritten Zahnrads gelagert ist, sodass durch die Lagerung des Lenkrings im Halterbügel, der auf der gemeinsamen Drehachse der Antriebsräder gelagert ist, der Eingriff des mit dem Lenkring verbundenen dritten Zahnrads mit den Antriebsrädern gesichert ist. Auf ein viertes Zahnrad kann daher vorteilhaft verzichtet werden.

Damit wird ein Lenkgetriebe geschaffen, das vorteilhaft wenig Bauraum erfordert.

Nach einer Ausführungsform des erfindungsgemäßen Lenkgetriebes kann der Haltebügel u-förmig mit einer Basis und zwei Schenkeln ausgebildet sein. Damit ist seine Geometrie in Bezug auf Raumeinnahme und Stabilität günstig.

Die Basis des Haltebügels kann eine Aufnahmeöffnung aufweisen, in der ein Lenkringstutzen, der an dem Lenkring ausgebildet ist, drehbar gelagert ist. Ferner kann jeder Schenkel ein Lagerauge aufweisen, mit dem der Haltebügel drehbar um die gemeinsame Drehachse der Antriebsräder gelagert ist. Beispielsweise kann der Haltebügel auf einem jeweiligen Achsstummel der Antriebsräder gelagert sein, der mit einer Antriebswelle des jeweiligen Motors gekoppelt ist. Die Antriebsräder können auch ohne Achsstummel mit einer Antriebswelle gekoppelt sein, sodass der Haltebügel auf einem Achsabschnitt der jeweiligen Antriebswelle angeordnet sein kann. Alternativ, wenn die Antriebsräder nicht direkt von einer Antriebswelle angetrieben werden, sondern drehbar auf einer Lagerachse angeordnet sind, kann der Haltebügel drehbar auf einem Achsabschnitt der jeweiligen Lagerachse der Antriebsräder angeordnet sein.

Gemäß einer weiteren Ausführungsform des erfindungsgemäßen Lenkgetriebes kann der Lenkringstutzen zylindrisch geformt sein und über ein Lenkringlager in einem zylindrischen Abschnitt der Aufnahmeöffnung gelagert sein.

Ferner kann nach einer weiteren Ausführungsform des erfindungsgemäßen Lenkgetriebes vorsehen, dass das dritte Zahnrad an dem Lenkring diametral zur Basis des Haltebügels vorliegt. Um hierbei den Eingriff des dritten Zahnrads mit den Antriebsrädern zu sichern, ist in einer weiteren Ausführungsform des erfindungsgemäßen Lenkgetriebes ein Befestigungselement vorgesehen, das eine Wirkverbindung zwischen dem Haltebügel und dem Lenkring bereitstellt und dazu ausgebildet ist, den Lenkring in Richtung der Basis des Haltebügels und damit das mit dem Lenkring verbundene dritte Zahnrad in Eingriff mit den Antriebsrädern zu ziehen. Auf diese Weise lässt sich sämtliches Spiel aus dem Eingriff zwischen dem dritten Zahnrad und den Antriebsrädern nehmen.

Das Befestigungselement zur Bereitstellung der Wirkverbindung des diametral zum dritten Zahnrad angeordneten Haltebügels mit dem Lenkring kann eine Schraube sein, die in den Lenkringstutzen in der Drehachse des dritten Zahnrads eingezogen ist und so das Lenkringlager und den Lenkring axial fixiert.

Gemäß einer alternativen, sehr vorteilhaften Ausführungsform liegen das dritte Zahnrad und die Basis des Haltebügels auf der gleichen Seite des Lenkrings vor, sodass der Eingriff des dritten Zahnrads mit den Antriebsrädern dadurch gesichert wird, dass die Basis des Haltebügels auf den Lenkring drückt. Vorteilhaft kann hierbei zum einen auf ein zusätzliches Befestigungselement verzichtet werden, und zum anderen wird durch die einseitige Anordnung von Haltebügelbasis und drittem Zahnrad am Lenkring ein kompakterer Aufbau erreicht bzw. Bauraum auf der anderen Seite des Lenkrings frei, der beispielsweise zur Unterbringung von anderen Komponenten des Lenkgetriebes genutzt werden kann.

Weitere Ausführungsformen eines erfindungsgemäßen Lenkgetriebes beziehen sich darauf, dass der Lenkring einstückig mit dem dritten Zahnrad ausgebildet sein kann, und / oder die Antriebsräder jeweils zumindest einen Kegelradkranzabschnitt zum Eingriff mit zumindest einem Kegelradkranzabschnitt des dritten Zahnrads aufweisen. Hierbei können die Kegelradkranzabschnitte der Antriebsräder und der Kegelradkranzabschnitt des dritten Zahnrads als vollumfängliche Kegelkränze ausgebildet sein oder bevorzugt - insbesondere in der Ausführung mit der einseitigen Anordnung von Haltebügelbasis und drittem Zahnrad - sich über einen ersten vorbestimmbaren umfänglichen Abschnitt des jeweiligen Antriebsrads bzw. über vorbestimmbare umfängliche Abschnitte des dritten Zahnrad erstrecken, die den Eingriff der Antriebsräder mit dem dritten Zahnrad in einem vorbestimmbaren Bewegungsumfang des Lenkrings bereitstellen.

Da der Lenkring für die bestimmungsgemäße Auslenkung einer Taumelscheibe in einem chirurgischen Instrument keine volle Drehung erfährt, sondern nur um einen Bruchteil einer ganzen Umdrehung, beispielsweise in einem Bereich von 30 ° bis 60 °, z. B. 45 °, in alle Raumrichtungen geschwenkt wird, kommt auch nur ein entsprechender Teil der Zähne eines vollumfänglichen Zahnkranzes der Antriebsräder überhaupt jemals in Eingriff mit dem dritten Zahnrad. Auf das somit nicht zum Eingriff mit dem dritten Zahnrad genutzte Segment am Umfang der Antriebsräder kann daher verzichtet werden. Entsprechend ist es auch möglich, dass das dritte Zahnrad anstelle eines umfänglichen Zahnkranzes nur die jeweils zum Eingriff mit den Antriebsrädern erforderliche Zahnkranzabschnitte aufweist.

Die Reduktion des Kegelradkranzes auf den jeweils zum Eingriff erforderlichen Kegelradkranzabschnitt ist vor allem in der Ausführung vorteilhaft, in der durch die Anordnung des dritten Zahnrads auf derselben Seite wie die Basis des Halterbügels, Bauraum auf der anderen Seite des Lenkrings frei wird. Denn durch den Verzicht auf die nicht benötigte Zahnung wird zusätzlich Bauraum frei, der dazu genutzt werden kann, weitere Komponenten, beispielsweise eine Getriebevorrichtung zur Übertragung der Stellbewegungen eines Motors auf ein Antriebsrad, unterzubringen.

So ist in einer Ausführungsform des erfindungsgemäßen Lenkgetriebes zwar vorgesehen, dass jedes Antriebsrad von dem zugeordneten Motor direkt über jeweils eine Antriebswelle betätigbar ist, deren Antriebsachse der gemeinsamen Drehachse der zwei Antriebsräder entspricht, allerdings beziehen sich dazu alternative Ausführungsformen darauf, dass jedes Antriebsrad von dem zugeordneten Motor über jeweils eine Antriebswelle und jeweils eine Getriebevorrichtung betätigbar ist, wobei die Antriebsachsen der Antriebswellen vorzugsweise auch parallel oder senkrecht zu der gemeinsamen Drehachse der zwei Antriebsräder verlaufen können, sodass eine platzsparende Unterbringung der Motoren, z. B. näher an der Längsachse ermöglicht wird.

Ausführungsformen eines erfindungsgemäßen Lenkgetriebes umfassen dabei ferner ein Ritzel oder eine Schneckenwelle oder ein Antriebskegelrad als Getriebevorrichtung, wobei die Antriebsräder jeweils zumindest einen entsprechenden Antriebskranzabschnitt zum Eingriff mit der jeweiligen Getriebevorrichtung aufweisen. D. h. dass ein Antriebskranzabschnitt zum Eingriff mit einem Ritzel als Getriebevorrichtung als Zahnkranz ausgebildet ist, wobei die Antriebsachsen beider Antriebswellen parallel zu der gemeinsamen Drehachse der zwei Antriebsräder verlaufen.

Ist die Getriebevorrichtung eine Schneckenwelle, so ist der Antriebskranzabschnitt als Schneckenradkranzabschnitt zum Eingriff mit der jeweiligen Schneckenwelle ausgebildet, wobei die Antriebsachsen beider Antriebswellen senkrecht zu der gemeinsamen Drehachse der zwei Antriebsräder verlaufen. Und im Falle eines Antriebskegelrads als Getriebevorrichtung ist der zumindest eine Antriebskranzabschnitt als Kegelradkranzabschnitt zum Eingriff mit dem jeweiligen Antriebskegelrad ausgebildet, wobei die Antriebsachsen beider Antriebswellen senkrecht zu der gemeinsamen Drehachse der zwei Antriebsräder verlaufen.

Zur Ausbildung der Antriebsräder mit dem Kegelradkranz(abschnitt) zum Eingriff mit dem dritten Zahnrad und mit den Antriebskranzabschnitten zum Eingriff mit einer jeweiligen Getriebevorrichtung können die Antriebsräder in einer Ausführungsform des erfindungsgemäßen Lenkgetriebes als Doppelräder ausgebildet sein, wobei der jeweilige Kegelradkranzabschnitt axial benachbart zu dem Antriebskranzabschnitt angeordnet ist und die Kegelradkranzabschnitte der beiden Doppelräder zum Eingriff mit dem dritten Zahnrad einander zugewandt sind.

Eine dazu alternative Ausführungsform, bei der sich der Kegelradkranzabschnitt nur über den zum Eingriff mit dem dritten Zahnrad erforderlichen ersten umfänglichen Abschnitt des jeweiligen Antriebsrads erstreckt, sieht vor, dass sich der jeweilige Antriebskranzabschnitt zum Eingriff mit der jeweiligen Getriebevorrichtung (Ritzel, Schneckenwelle, Antriebskegelrad) in einem vorbestimmbaren Bewegungsumfang des Lenkrings über einen zweiten vorbestimmbaren umfänglichen Abschnitt des jeweiligen Antriebsrads erstreckt, der keine Überschneidung mit dem jeweiligen Kegelradkranzabschnitt aufweist.

Eine alternative Getriebevorrichtung einer weiteren Ausführungsform eines erfindungsgemäßen Lenkgetriebes ist eine Zahnstangengetriebevorrichtung, die ein Antriebsritzel, eine Zahnstange mit einer Stirnverzahnung und mit einer Seitenzahnung sowie ein Übertragungsritzel aufweist. Dabei ist das Antriebsritzel über die Antriebswelle von dem jeweils zugeordneten Motor betätigbar und steht mit der Seitenzahnung der Zahnstange in Eingriff. Das Übertragungsritzel steht mit der Stirnzahnung der Zahnstange in Eingriff und ist über eine Abtriebswelle mit dem Antriebsrad verbunden, wobei die Antriebsachsen senkrecht zu der gemeinsamen der zwei Antriebsräder verlaufen.

Gemäß einer weiteren alternativen Ausführungsform eines erfindungsgemäßen Lenkgetriebes kann die Getriebevorrichtung eine Spindel oder Riemenscheibe und ein Zugmittel aufweisen, wobei die Antriebsräder als Doppelräder ausgebildet sind, bei denen der Antriebskranz als Zugmittelscheibe bzw. Riemenscheibe ausgebildet ist. Das Zugmittel stellt eine Wirkverbindung zwischen der Spindel oder Riemenscheibe, die an der Antriebswelle angeordnet ist, und dem als Zugmittelscheibe oder Riemenscheibe ausgebildeten Antriebskranz bereit. Dabei können die Antriebsachsen der Getriebevorrichtung mit Spindel aufgrund der Möglichkeit der Zugmittelumlenkung in beliebiger Orientierung in Bezug zu der gemeinsamen Drehachse verlaufen, während die Antriebsachsen der Getriebevorrichtung mit Riemenscheibe parallel zu der gemeinsamen Drehachse der zwei Antriebsräder verlaufen.

Eine erste Ausführungsform eines erfindungsgemäßen chirurgischen Instruments, das einen Schaft, eine am proximalen Ende des Schaftes angeordnete Betätigungseinheit und ein am distalen Ende des Schaftes angeordnetes Werkzeug mit einer mittels einer distalen Abwinkelungsmechanik abwinkelbaren Werkzeugspitze aufweist, die durch eine mittels zweier Antriebe räumlich ausrichtbare Taumelscheibe steuerbar ist, bezieht sich darauf, dass das chirurgische Instrument ein erfindungsgemäßes Lenkgetriebe zur räumlichen Ausrichtung der Taumelscheibe aufweist.

In weiteren Ausführungsformen des erfindungsgemäßen chirurgischen Instruments kann vorgesehen sein, dass die Taumelscheibe kardanisch mit einer koaxial zu einer Längsachse des Schaftes verlaufenden Hauptwelle gekoppelt ist, und / oder das Betätigungselement axial verschiebbar in dem Schaft gelagert ist und proximalseitig mit der Betätigungseinheit in Wirkverbindung steht. Ferner kann die distale Abwinkelungsmechanik der abwinkelbaren Werkzeugspitze aus an dem distalen Ende des Schaftes angeordneten Schwenkgliedern bestehen, die über in Längsrichtung des Schaftes verlaufende Lenkdrähte mit der Taumelscheibe des Lenkgetriebes verbunden sind. Zudem können die Lenkdrähte an der Taumelscheibe lösbar, beispielsweise mittels einer Klemmverbindung klemmend befestigt sein. Dazu alternative Befestigungsformen der Lenkdrähte an der Taumelscheibe umfassen beispielsweise Schweißen oder Crimpen. Ferner kann distalseitig vor der räumlich verstellbaren Scheibe eine Fächerscheibe auf der Hauptwelle angeordnet sein, die den radialen Abstand der Lenkdrähte von der Längsachse des Schaftes vergrößert, wobei die Lenkdrähte zwischen der Fächerscheibe und der Taumelscheibe annährend parallel zueinander verlaufen. Aber auch ohne Fächerscheibe ist der radiale Abstand der Lenkdrähte von der Längsachse des Schaftes an der Taumelscheibe größer als am proximalen Ende des Schaftes, wenn sich die Lenkdrähte von dem proximalen Ende des Schaftes direkt zu der Taumelscheibe erstrecken, sodass die Lenkdrähte unter einem von 90 ° abweichenden Winkel zur Taumelscheibe verlaufen.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich schematische Darstellungen von Ausführungsformen der Erfindung. In den Zeichnungen ist ein Ausführungsbeispiel der Erfindung dargestellt. Die Zeichnungen und die Beschreibung enthalten zahlreiche Merkmale in Kombination. Der Fachmann wird die Merkmale zweckmäßigerweise auch einzeln betrachten und zu sinnvollen weiteren Kombinationen unter Einhaltung des Geltungsbereichs der Ansprüche zusammenfassen.

Dabei zeigen:
- Fig. 1: eine schematische perspektivische Seitenansicht eines chirurgischen Instruments,
- Fig. 2: eine perspektivische Detailansicht eines motorisierten Antriebs mit einem Taumelscheiben-Lenkgetriebe aus dem Stand der Technik,
- Fig. 3: eine perspektivische Detailansicht eines motorisierten Antriebs mit einem Taumelscheiben-Lenkgetriebe nach einer erfindungsgemäßen Ausführungsform,
- Fig. 4: eine Seitenschnittdetailansicht des Lenkgetriebes aus Fig. 3,
- Fig. 5: eine perspektivische Detailansicht eines motorisierten Antriebs mit einem Taumelscheiben-Lenkgetriebe nach einer weiteren erfindungsgemäßen Ausführungsform,
- Fig. 6: eine Seitenschnittansicht eines motorisierten Antriebs mit einem Taumelscheiben-Lenkgetriebe nach einer weiteren erfindungsgemäßen Ausführungsform ähnlich Fig. 4 durch eine von Achsen A und C aufgespannte Ebene,
- Fig. 7: eine perspektivische Ansicht eines Haltebügels,
- Fig. 8: eine perspektivische Detailansicht eines motorisierten Antriebs mit einem Taumelscheiben-Lenkgetriebe nach einer weiteren erfindungsgemäßen Ausführungsform mit Stirnradgetriebe,
- Fig. 9: eine perspektivische Detailansicht eines motorisierten Antriebs mit einem Taumelscheiben-Lenkgetriebe nach einer weiteren erfindungsgemäßen Ausführungsform mit Schneckenradgetriebe,
- Fig. 10: eine perspektivische Detailansicht eines motorisierten Antriebs mit einem Taumelscheiben-Lenkgetriebe nach einer weiteren erfindungsgemäßen Ausführungsform mit Zahnstangengetriebe,
- Fig. 11: eine perspektivische Detailansicht eines motorisierten Antriebs mit einem Taumelscheiben-Lenkgetriebe nach einer weiteren erfindungsgemäßen Ausführungsform mit Kegelradgetriebe,
- Fig. 12: eine perspektivische Detailansicht eines motorisierten Antriebs mit einem Taumelscheiben-Lenkgetriebe nach einer weiteren erfindungsgemäßen Ausführungsform mit Spindelgetriebe,
- Fig. 13: eine perspektivische Detailansicht eines motorisierten Antriebs mit einem Taumelscheiben-Lenkgetriebe nach einer weiteren erfindungsgemäßen Ausführungsform mit Riemengetriebe.

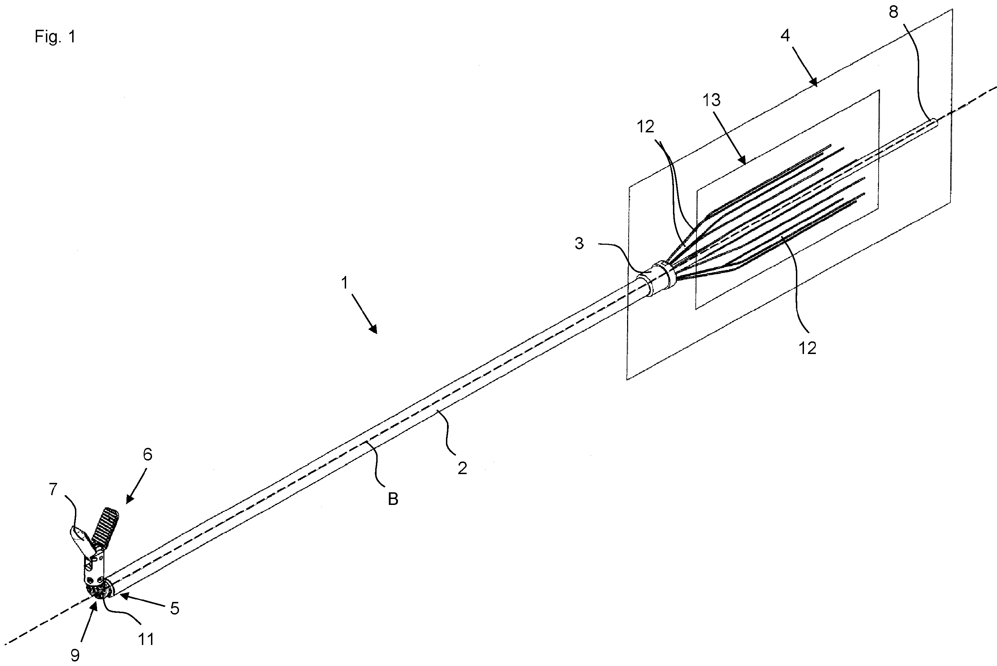

Fig. 1 zeigt schematisch ein chirurgisches Instrument 1 mit einem hohlen Schaft 2, einer am proximalen Ende 3 des Schaftes 2 angeordneten, nur schematisch dargestellten Betätigungseinheit 4 und einer am distalen Ende 5 des Schaftes 2 angeordneten Werkzeugspitze 6 mit einem Werkzeug 7. Das Werkzeug 7 ist über ein axial verschiebbar im Schaft 2 gelagertes Betätigungselement 8 betätigbar, das proximalseitig mit der Betätigungseinheit 4 in Wirkverbindung steht. Bei der Betätigungseinheit 4 kann es sich um eine manuell betätigbare Handhabe oder aber um eine für den robotischen Einsatz ausgelegte, also auch ohne manuelles Zutun betätigbare Baueinheit handeln - was vorteilhaft für die Reproduzierbarkeit der Betätigung ist. Bei dem Werkzeug 7 der Werkzeugspitze 6 kann es sich beispielsweise um ein mit Maulteilen versehenes Werkzeug, wie in Fig. 1 dargestellt, oder aber um ein Endoskop, einen Applikator oder dergleichen handeln. Die Werkzeugspitze 6 ist über einen Gelenkmechanismus 9 relativ zur Längsachse B des Schaftes 2 verschwenkbar, wobei der Gelenkmechanismus 9 aus am distalen Ende des Schaftes 5 angeordneten Schwenkgliedern 11 besteht, die über in Längsrichtung des Schaftes 2 verlaufende Lenkdrähte 12 so mit einem am proximalen Ende 3 des Schaftes 2 angeordneten Antrieb 13 verbunden sind, dass eine Bewegung des proximalseitigen Antriebs 13 eine entsprechende relative Bewegung der distalseitigen Schwenkglieder 11 und somit ein Verschwenken der Werkzeugspitze 6 verursacht. Auch wenn voranstehend und nachfolgend nur der Begriff Lenkdrähte 12 verwendet wird, können funktional auch Lenkseile verwendet werden, weshalb der verwendete Begriff Lenkdrähte 12 synonym auch als Lenkseil zu lesen und zu verstehen ist.

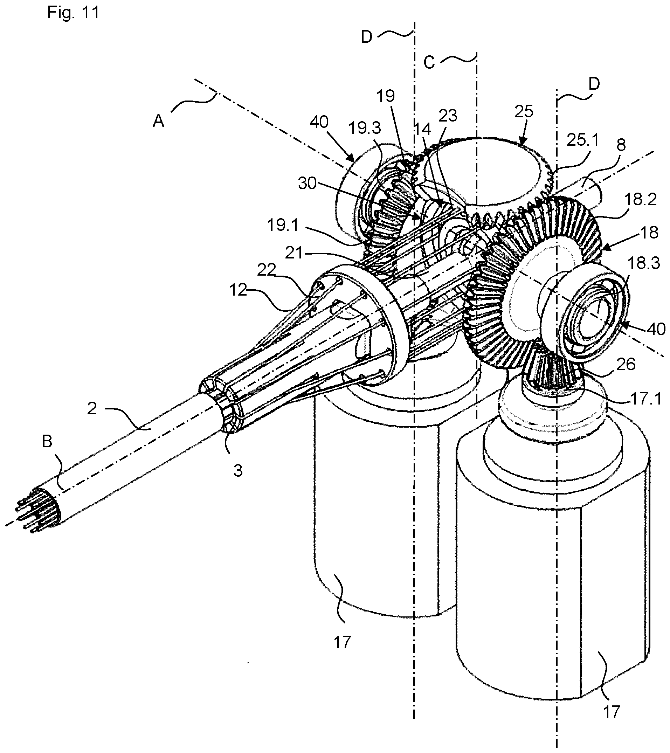

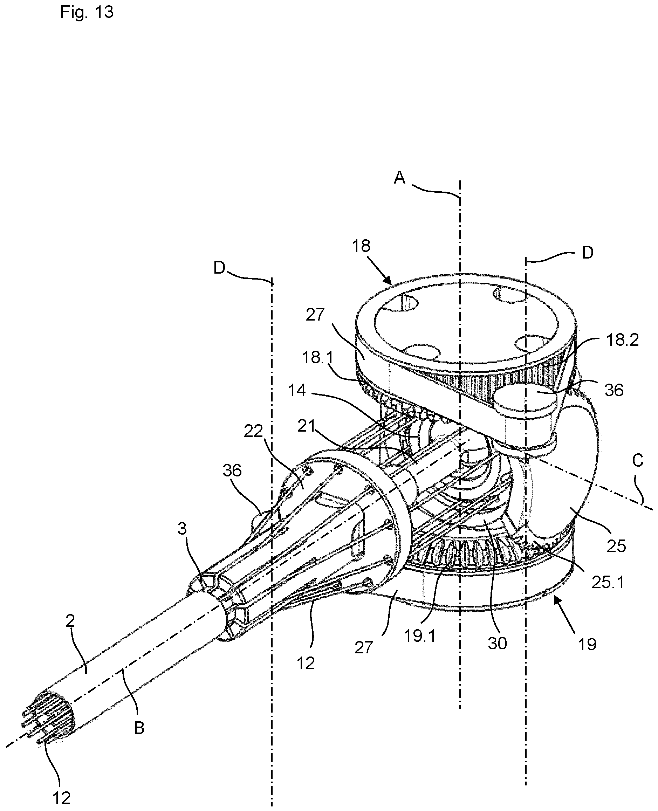

Das axialverschiebbar im Schaft 2 gelagerte Betätigungselement 8 zum Betätigen des beispielsweise aus zwei Maulteilen bestehenden Werkzeugs 7 ist bei den dargestellten Ausführungsformen als Zug- / Schubstange ausgebildet. Der Antrieb 13 für die Lenkdrähte 12 kann bei dem erfindungsgemäßen medizinischen Instrument 1 vorzugsweise als motorisierter Antrieb 13 ausgebildet sein, der eine räumlich verstellbare Taumelscheibe 14 aufweist, die kardanisch mit einer koaxial zum Schaft 2 verlaufenden Hauptwelle 21 gekoppelt ist, um die Taumelscheibe 14 dreidimensional relativ zur Längsachse B des Schaftes 2 zu verlagern. Dabei sind die Lenkdrähte 12 so an der Taumelscheibe 14 gelagert bzw. befestigt (vgl. Fig. 11, 13), dass eine über den motorisierten Antrieb 13 bewirkte Verlagerung der Taumelscheibe 14 über die Lenkdrähte 12 ein Verschwenken der Werkzeugspitze 6 bewirkt. Die Anzahl der zu verwendenden Lenkdrähte 12 für einen motorisierten Antrieb 13 ist frei wählbar. Durch eine optionale Fächerscheibe 22 werden die parallel zur Längsachse B des Schaftes 2 verlaufenden Lenkdrähte 12 der Taumelscheibe 14 zugeführt. Zum Festlegen der Lenkdrähte 12 an der Taumelscheibe 14 sind in der Taumelscheibe 14 Durchgangsbohrungen 23 für jeden Lenkdraht 12 ausgebildet, die beispielsweise über radial in die Taumelscheibe eingebrachte Madenschrauben oder durch eine proximalseitig hinter der Taumelscheibe 14 angeordnete Klemmscheibe kraftschlüssig mit der Taumelscheibe 14 verbunden und fixiert sein können.

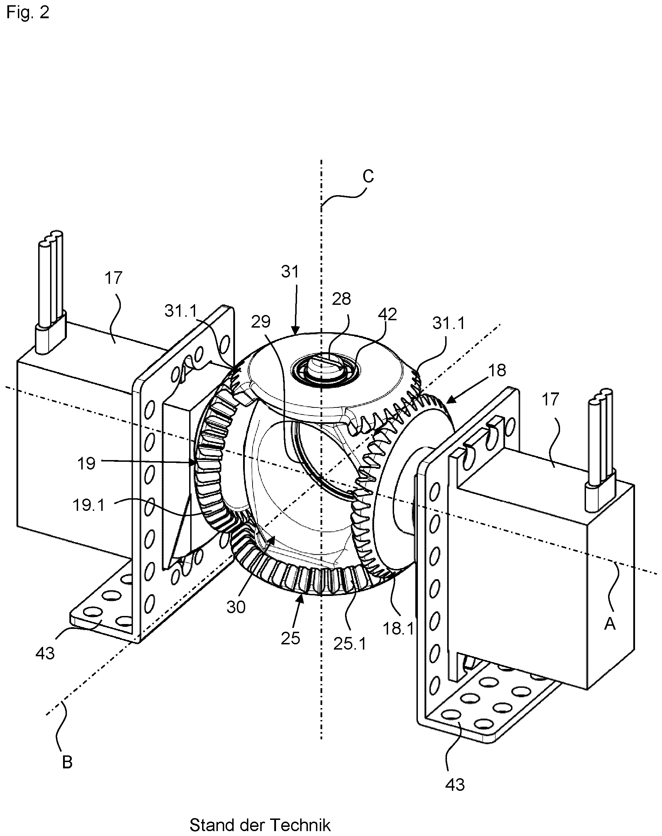

Ein aus dem Stand der Technik bekannter motorisierter Antrieb ist in Fig. 2 dargestellt, wobei die dort nicht dargestellte Taumelscheibe über einen Lagerring 29 in dem Lenkring 30 gelagert ist, um eine Rotation der Taumelscheibe 14 mit der Hauptwelle 21 (vgl. Fig. 11, 13), die sich entlang der Längsachse B des Schaftes 2 erstreckt, zu ermöglichen. Dabei ist die in dem Lenkring 30 gelagerte Taumelscheibe zwischen zwei um 180 ° zueinander versetzt angeordneten Antriebsrädern 18, 19 angeordnet ist, die von jeweils einem Motor 17 betätigt werden, der mittels einer Halterung 43 im chirurgischen Instrument 1 montierbar ist. Die Antriebswellen der Antriebsräder 18, 19 liegen auf einer gemeinsamen Drehachse A, die senkrecht zur Längsachse B des in Fig. 2 nicht dargestellten Schaftes 2 liegt. Die Antriebsräder 18, 19 sind als Kegelräder ausgebildet und weisen jeweils einen Kegelradkranz 18.1, 19.1 auf. Die beiden Antriebsräder 18, 19 sind mit einem dritten Zahnrad 25 gekoppelt, das ebenfalls als Kegelrad mit einem Kegelradkranz 25.1 ausgebildet ist und mit den beiden Kegelradkränzen 18.1, 19.1 der Antriebsräder 18, 19 in Eingriff steht, sodass die Drehachse C des dritten Zahnrads 25 die gemeinsame Drehachse A der Antriebsräder 18, 19 sowie die Längsachse B des Schaftes 2 schneidet. Das dritte Zahnrad 25 ist drehfest mit dem Lenkring 30 verbunden, sodass jede Bewegung der beiden Antriebsräder 18, 19 durch die drei miteinander kämmenden Kegelräder 18, 19 und 25 direkt auf die über den Lenkring 30 mit dem dritten Zahnrad 25 gekoppelte Taumelscheibe 14 übertragen wird, was eine direkte Betätigung der Lenkdrähte 12 bewirkt. Zur Sicherung des Lenkrings 30 ist auf der Drehachse C des dritten Zahnrads 25 gegenüber liegend zum dritten Zahnrad 25 ein viertes Zahnrad 31 quasi als Deckelrad angeordnet, das ebenfalls als Kegelrad mit zwei Kegelradkranzabschnitten 31.1 ausgebildet ist, die mit den Kegelradkränzen 18.1, 19.1 der beiden Antriebsräder 18 und 19 in Eingriff stehen. So wird die durch die Antriebsräder 18, 19 und Zahnrad 25 gebildete Verzahnungskette zu einem geschlossenen Verzahnungsring geschlossen, der sicherstellt, dass alle Verzahnungen in Eingriff bleiben, sodass eine gleichmäßig umlaufende Kraftverteilung gewährleistet ist. Über eine Schraube 28, die das vierte Zahnrad 31 zum Lenkring 30 zieht, lässt sich sämtliches Spiel aus der Verzahnungskette der vier Zahnräder 18, 19, 25, 31 nehmen. Dabei ist der drehfest mit dem dritten Zahnrad 25 gekoppelte Lenkring 30 durch eine Lagerung mittels Lagerring 42 frei in Bezug auf das vierte Zahnrad 31 drehbar, so dass eine Rotation des vierten Zahnrads 31 um seine Drehachse C keine Verdrehung des Lenkrings 30 und somit der Taumelscheibe bewirkt.

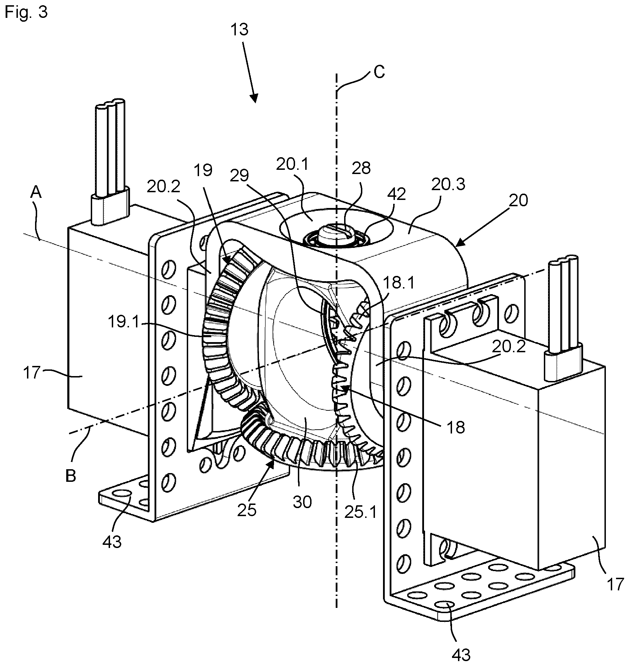

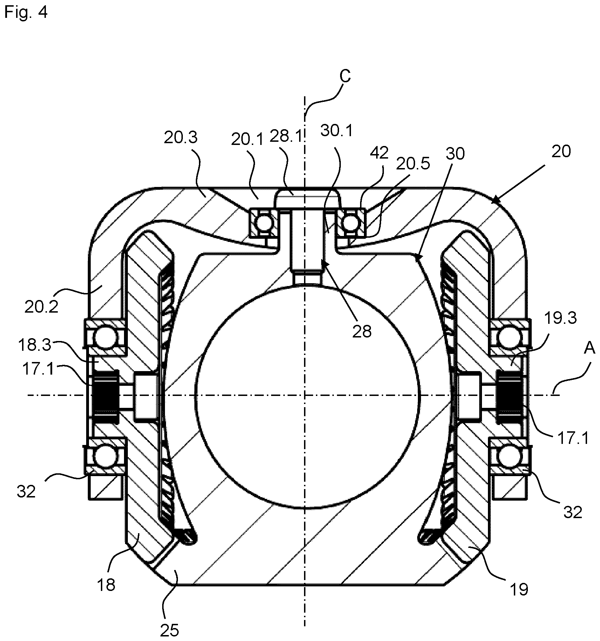

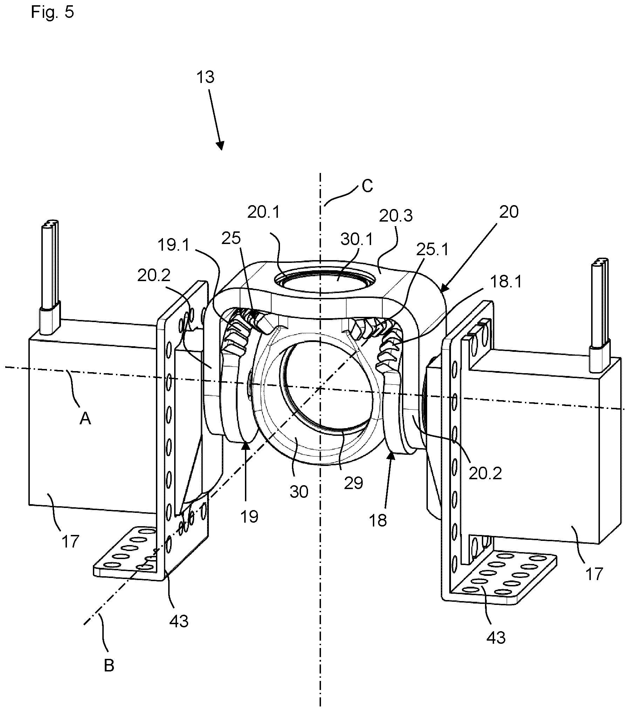

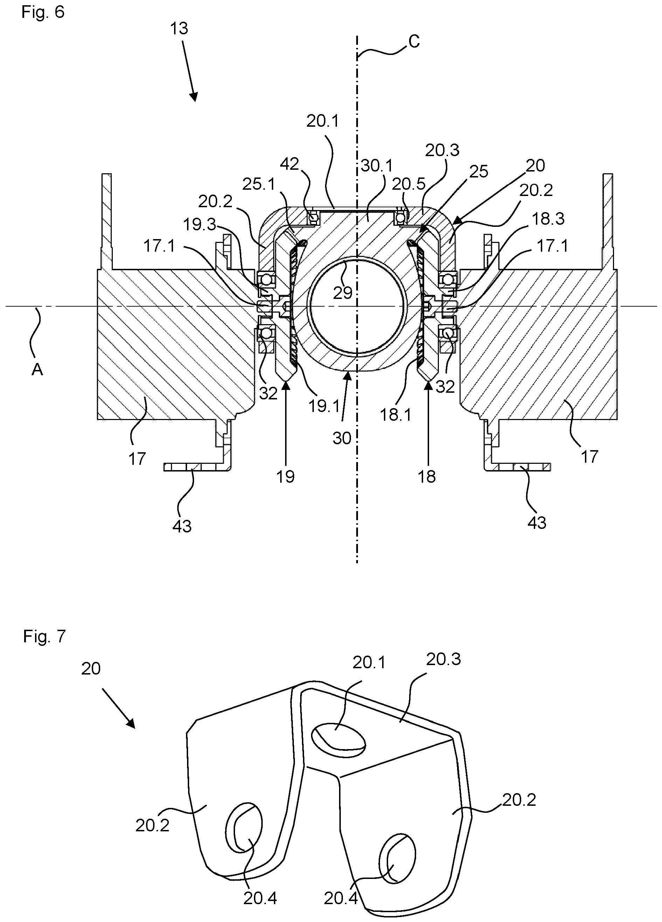

Fig. 3 zeigt ein erfindungsgemäßes Beispiel für ein Lenkgetriebe 13, das sich von dem motorisierten Antrieb aus dem Stand der Technik gemäß Fig. 2 dadurch unterscheidet, dass auf das vierte Zahnrad 31 verzichtet wird. Um dennoch den Eingriff der Antriebsräder 18, 19 mit dem dritten Zahnrad 25 und den Lenkring 30 zu sichern, weist das erfindungsgemäße Lenkgetriebe 13 einen Haltebügel 20 als eine Eingriffssicherungsvorrichtung auf, die im Beispiel der Fig. 3 bis 6 durch einen Haltebügel 20 gebildet wird, der zusätzlich in Fig. 7 schematisch dargestellt ist. Der Haltebügel 20 ist u-förmig mit zwei sich von einer Basis 20.3 erstreckenden Schenkeln 20.2 ausgebildet. Die in Fig. 5 und 6 gezeigte Ausführungsform unterscheidet sich durch die Anordnung des dritten Zahnrads 25 von der Ausführungsform in Fig. 3 und 4: Dort liegt die Basis 20.3 des Haltebügels 20 in Bezug auf den Lenkring 30 diametral zu dem dritten Zahnrad 25, während sich in Fig. 5 und 6 die Basis 20.3 des Haltebügels 20 und das dritte Zahnrad 25 auf derselben Seite des Lenkrings 30 befinden. Dabei ist die in Fig. 5 und 6 gezeigte Ausführungsform bevorzugt, die gegenüber dem Stand der Technik platzsparender aufgebaut ist und auf einer Seite des Lenkrings 30 - in der Darstellung von Fig. 5 und 6 unter dem Lenkring 30 - freien Bauraum schafft.

Der Haltebügel 20 in den Beispielen der Fig. 3 bis 6 ist an den Schenkeln 20.2 über entsprechende in den Lageraugen 20.4 aufgenommene Lager 32 drehbar auf den Achsstummeln 18.3, 19.3 der Antriebsräder 18, 19 gelagert, sodass das fest mit dem Lenkring 30 verbundene dritte Zahnrad 25 ständig im Eingriff mit den Antriebsrädern 18, 19 steht.

Selbstverständlich kann in davon abweichenden Modifikationen der Haltebügel auf einem von einem Achsstummel abweichenden Achsabschnitt einer Antriebswelle oder Lagerachse der Antriebsräder angeordnet sein. In der Basis 20.3 ist eine Aufnahmeöffnung 20.1 vorgesehen, um einen hier zylindrischen Lenkringstutzen 30.1 des Lenkrings 30 mittels eines Lagerrings 42 frei in Bezug auf den Haltebügel 20 drehbar zu lagern. Dieser weist in der Aufnahmeöffnung 20.1 einen Lagersitz 20.5 für das Lenkringlager 42 auf, dessen Ausrichtung davon abhängt, ob das dritte Zahnrad 25 diametral zum Haltebügel 20 oder auf derselben Seite wie der Haltebügel 20 angeordnet ist. Bei der gleichseitigen Anordnung von Haltebügel 20 und drittem Zahnrad 25 ist, wie in Fig. 6 zu sehen, der Lagersitz 20.5 und das Lenkringlager 42 auf einer dem Lenkring 30 zugewandten Seite der Basis 20.3 um bzw. in der Aufnahmeöffnung 20.1 ausgebildet bzw. angeordnet. Bei der diametralen Anordnung des Haltebügels 20 zum dritten Zahnrad 25, die in Fig. 4 dargestellt ist, ist der Lagersitz 20.5 und damit das Lenkringlager 42 auf einer von dem Lenkring 30 abgewandten Seite der Basis 20.3 um bzw. in der Aufnahmeöffnung 20.1 ausgebildet bzw. angeordnet.

In der Ausführungsform entsprechend Fig. 3 wird der Lenkring 30 mit einem Befestigungselement 28 wie der dargestellten Schraube in Richtung der Basis 20.3 des Haltebügels 20 gezogen, sodass das mit dem Lenkring 30 verbundene dritte Zahnrad 25 ebenfalls nach oben gezogen wird, um einen zumindest spielarmen, wenn möglich spielfreien Eingriff des Zahnrads 25, das als Kegelrad mit Kegelradkranz 25.1 ausgebildet ist, mit den Kegelradkränzen 18.1, 19.1 der Antriebsräder 18, 19 zu sichern. Der Kopf 28.1 der Schraube 28, die in den Lenkringstutzen 30.1 eingezogen ist, sorgt dabei gleichzeitig für die axiale Fixierung des Lenkringlagers 42 und des Lenkrings 30 in Bezug auf Drehachse C.

Im Beispiel von Fig. 5 und 6 wird das dritte Zahnrad 25, das in diesem Beispiel ferner einstückig mit dem Lenkring 30 ausgebildet ist, von dem Haltebügel 20, der hier ebenfalls über die Schenkel 20.2 auf den Achsstummeln 18.3, 19.3 gelagert ist, gegen die Antriebsräder 18, 19 gedrückt, um den Eingriff zu sichern. Daher kann hier auf ein Befestigungselement wie Schraube 28 verzichtet werden, sodass sich der Lenkringstutzen 30.1 nur zur drehbaren Lagerung mittels Lager 42 in die Aufnahmeöffnung 20.1 des Haltebügels 20 erstreckt. Das Beispiel in Fig. 5 und 6 zeigt ferner, dass die Antriebsräder 18, 19 und das dritte Zahnrad 25 anstelle eines umfänglichen Kegelradkranzes 18.1, 19.1 bzw. Kegelradkranzes 25.1 wie in Fig. 3 lediglich Kegelradkranzabschnitte 18.1, 19.1 bzw. 25.1 in den für den Eingriff und zur Steuerung der Taumelscheibe erforderlichen Abschnitten aufweisen können. Die Antriebsräder 18, 19 weisen demnach jeweils nur noch einen Kegelradkranzabschnitt 18.1, 19.1 auf, der sich entlang eines Bogenabschnitts erstreckt, der für eine maximale Abwinkelung der Werkzeugspitze 6 von beispielsweise 90 ° erforderlich ist. Aufgrund der unterschiedlichen Teilkreise in der Abwinkelung an der Werkzeugspitze und in dem Lenkgetriebe kann der dort auszuführende Lenkwinkel deutlich geringer sein als die damit an der Werkzeugspitze erzielbare Abwinkelung. Um die Werkzeugspitze um 90 ° abzuwinkeln, kann im Lenkgetriebe ein Lenkwinkel von nur z. B. 30 ° ausreichend sein, weshalb nur ein Abschnitt der Verzahnung je im Eingriff ist und die Aussparungen möglich sind. Das dritte Zahnrad 25 weist entsprechend zum Eingriff mit den Antriebsrädern 18, 19 jeweils einen entsprechenden Kegelradkranzabschnitt 25.1 auf. Durch die Aussparung nicht erforderlicher Zähne wird die durch die einseitige Anordnung des dritten Zahnrads 25 und des Haltebügels 20 erreichte Platzeinsparung noch vergrößert.

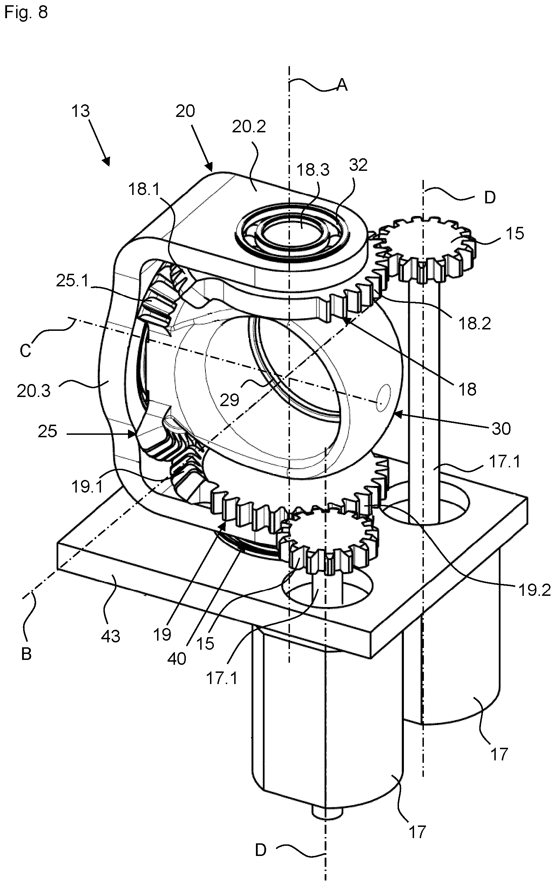

Der so gewonnene Bauraum lässt sich nutzen, um beispielsweise die Motoren 17, deren Antriebsachsen in Fig. 3 bis 6 auf der gemeinsamen Drehachse A der Antriebsräder 18, 19 liegen, anders bzw. platzsparender anordnen zu können und mit verschiedenen Getriebevorrichtungen die Antriebsbewegungen der Motoren auf die Antriebsräder 18, 19 übertragen zu können, indem die Antriebsachsen der Motoren 17 von der gemeinsamen Drehachse A abweichen können und beispielsweise senkrecht oder parallel dazu angeordnet werden können, wie in den nachfolgenden Beispielen anhand Fig. 8 bis 13 verdeutlicht ist.

Fig. 8 zeigt ein Beispiel eines erfindungsgemäßen Lenkgetriebes 13 mit einer Getriebevorrichtung zur Übertragung der Stellbewegungen von parallel angeordneten Motoren 17 auf die Antriebsräder 18, 19. Der Haltebügel 20 als Eingriffssicherungsvorrichtung ist in der gezeigten Darstellung an den Schenkeln 20.2 durch Lager 32 auf jeweils einem Achsstummel 18.3, 19.3 gelagert, der sich zudem zur weiteren Lagerung in einem Lager 40 in eine Halterung 43 erstrecken kann (in Fig. 8 nur einseitig angedeutet). Die hier dargestellte schematische Halterung 43 ist nur beispielhaft und in keiner Weise beschränkend zu verstehen. Um die Stellbewegungen der beiden Motoren 17, die hier auf der anderen Seite der Halterung 43 angeordnet sind, auf die Antriebsräder 18, 19 zu übertragen, ist jeweils ein Ritzel 15 vorgesehen, das über eine jeweilige Antriebswelle 17.1, die sich durch eine Öffnung in der Halterung 43 erstreckt, von dem zugeordneten Motor 17 angetrieben wird. Die parallele Anordnung der Motoren 17 nebeneinander bedingt hier unterschiedlich lange Antriebswellen 17.1, um den Eingriff der Ritzel 15 mit dem jeweiligen Antriebsrad 18, 19 bereitzustellen. Die Antriebswellen 17.1 definieren die Antriebsachsen D, die nicht nur parallel zueinander, sondern auch parallel zu der gemeinsamen Drehachse A der zwei Antriebsräder 18, 19 verlaufen. Auf diese Weise können die Antriebsachsen D relativ nahe an Längsachse B positioniert werden. Die Antriebsräder 18, 19 weisen zum Eingriff mit dem jeweiligen Ritzel 15 einen Antriebskranzabschnitt 18.2, 19.2 auf, der sich am Umfang des jeweiligen Antriebsrads 18, 19 über einen für die Stellbewegungen erforderlichen Abschnitt erstreckt, der sich von dem Umfangsabschnitt unterscheidet, an dem der jeweilige Kegelradkranzabschnitt 18.1, 19.1 zum Eingriff mit dem dritten Zahnrad 25 ausgebildet ist. Dies ist aufgrund des vorbestimmbaren beschränkten Bewegungsumfangs von Lenkring 30 und Taumelscheibe, die keine vollen Drehungen zur Abwinkelung der Werkzeugspitze 6 erfahren, möglich.

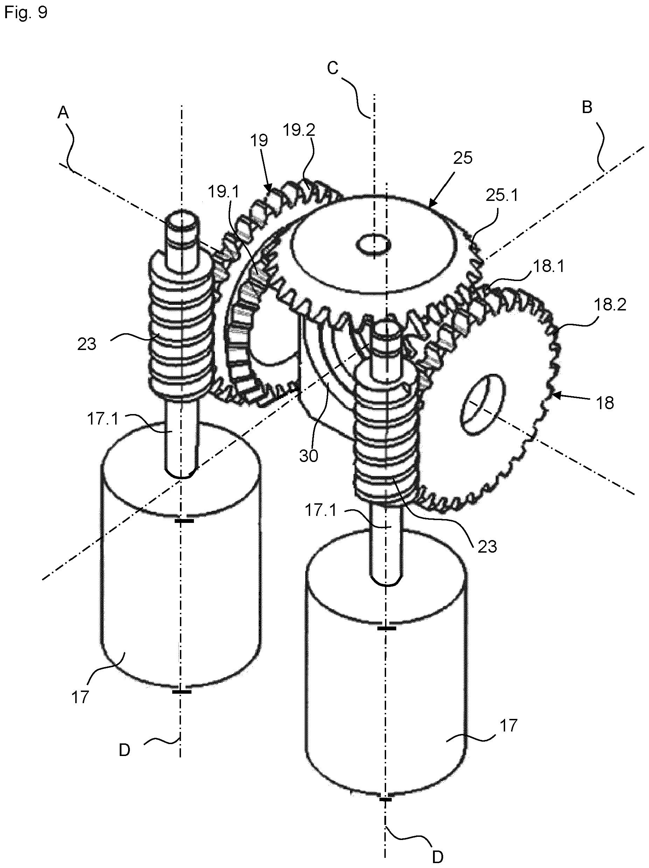

Da die Beispiele von Fig. 9 bis 13 unterschiedliche Getriebevorrichtungen verdeutlichen sollen, wird dort der Übersichtlichkeit wegen auf die Darstellung des Haltebügels als Eingriffssicherungsvorrichtung des erfindungsgemäßen Lenkgetriebes 13 verzichtet. Die dazu erforderliche Ergänzung mit einem Haltebügel entsprechend einem der Beispiele aus Fig. 3 bis 8 ergibt sich in naheliegender Weise.

Die Getriebevorrichtung eines erfindungsgemäßen Lenkgetriebes aus Fig. 9 weist zur Übertragung der Stellbewegungen der parallel angeordneten Motoren 17 auf die Antriebsräder 18, 19 jeweils eine Schneckenwelle 23 auf, die anders als im gezeigten Beispiel auch eine mehrgängige Schneckenwelle sein kann. Die Schneckenwellen 23 verlaufen an den Antriebswellen 17.1 entlang der jeweiligen Antriebsachse D und stehen jeweils mit einem als Schneckenrad ausgebildeten Antriebskranz 18.2, 19.2 der Antriebsräder 18, 19 in Eingriff, die in diesem Beispiel als Doppelräder ausgebildet sind. Dazu sind die Antriebskränze 18.2, 19.2 axial benachbart zu den Kegelradkränzen 18.1, 19.1 angeordnet, die einander zum Eingriff mit dem Kegelradkranz 25.1 des dritten Zahnrads 25 zugewandt sind. Die Doppelräder können einstückig gefertigt sein, oder aus jeweils einem mit einem Schneckenrad verbundenen Kegelrad bestehen. Im gezeigten Beispiel verlaufen die Antriebsachsen D nicht nur senkrecht zur gemeinsamen Drehachse A der Antriebsräder, sondern auch senkrecht zur Längsachse B. Es ist aber offensichtlich, dass die Antriebseinheiten aus Motor 17, Antriebswelle 17.1 und Schneckenwelle 23 - auch unabhängig voneinander - an einer beliebigen Position des Umfangs der Antriebskränze 18.2, 19.2 der Antriebsräder 18, 19 angeordnet werden können, sodass die Antriebsachsen D zwar immer senkrecht zur gemeinsamen Drehachse A, aber beliebig in Bezug auf die Längsachse B, beispielsweise auch parallel dazu, verlaufen können.

Abweichend von den in Fig. 8 und 9 dargestellten Beispielen kann ein erfindungsgemäßes Lenkgetriebe auch eine Getriebevorrichtung aufweisen, bei der die Ritzel 15 wie in Fig. 8 mit einem Antriebskranz 18.2, 19.2 eines als Doppelrad ausgebildeten Antriebsrads 18, 19 wie in Fig. 9 in Eingriff stehen. Die Ausführung mit Doppelrad ermöglicht dann auch hier die beliebige und unabhängige Anordnung der Antriebseinheiten aus Motor 17, Antriebswelle 17.1 und Ritzel 15 an einer beliebigen Position entlang dem Umfang der Antriebskränze 18.2, 19.2 der Antriebsräder 18, 19. Umgekehrt kann eine Schneckenwelle 23 wie in Fig. 9 mit einem Antriebskranzabschnitt 18.2, 19.2 eines Antriebsrads 18, 19 wie in Fig. 8, das entlang dem Umfang ferner einen Kegelkranzabschnitt 18.1, 19.1 aufweist, der sich nicht mit dem Antriebskranzabschnitt 18.2, 19.2 überschneidet, ebenfalls eine Getriebevorrichtung eines erfindungsgemäßen Lenkgetriebes 13 bilden.

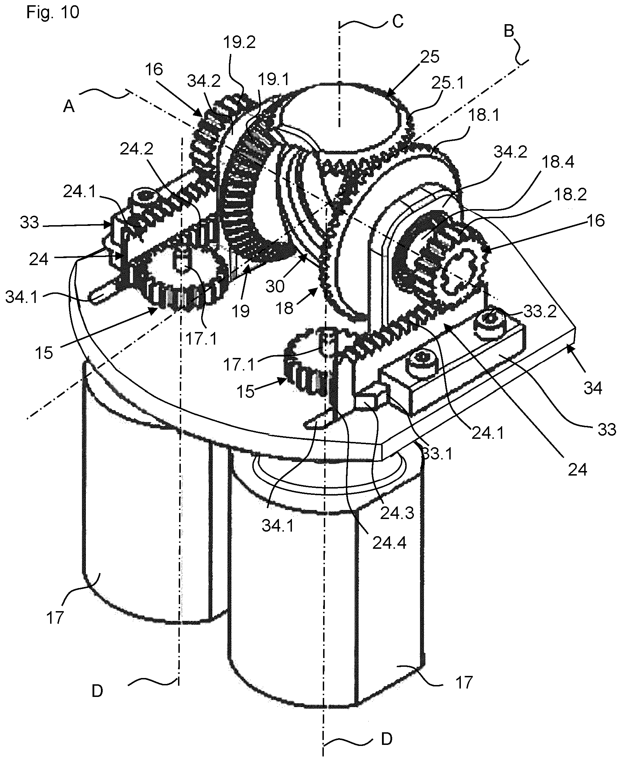

Ein weiteres Beispiel für eine alternative Getriebevorrichtung eines erfindungsgemäßen Lenkgetriebes 13 ist ein Zahnstangengetriebe, wie beispielhaft in Fig. 10 dargestellt ist.

Dabei ist die Lenkringeinheit aus dem Lenkring 30, dem damit verbundenen dritten Zahnrad 25 und den mit diesem in Eingriff stehenden Antriebsrädern 18, 19 auf einer Plattform 34 angeordnet. Dazu weist die Plattform 34 benachbart zu den zwei Antriebsrädern 18, 19 jeweils einen Lagerbügel 34.2 mit einem Lagerauge zur Aufnahme und Lagerung einer dem jeweiligen Antriebsrad 18, 19 zugeordneten Abtriebswelle 18.4 auf, die entlang der gemeinsamen Drehachse A verläuft. Die Abtriebswellen 18.4, von denen in Fig. 10 aufgrund der Perspektive nur die Abtriebswelle 18.4 des in der Darstellung vorderen Antriebsrads 18 zu sehen ist, verbinden das jeweilige Antriebsrad 18, 19 mit jeweils einem Übertragungsritzel 16, dessen Antriebskranz 18.2, 19.2 mit der Stirnzahnung 24.1 einer Zahnstange 24 kämmt. Diese ist längsbeweglich mit einem Führungsabschnitt 24.4 in einer Führungsnut 34.1 angeordnet, die in der Plattform 34 senkrecht zu der gemeinsamen Drehachse A und parallel zur Längsachse B ausgebildet ist. Die Zahnstange 24 weist ferner eine Seitenzahnung 24.2 auf, die mit einem Antriebsritzel 15 in Eingriff steht, das über die Antriebswelle 17.1 des Motors 17 betätigbar ist. Die Motoren 17 sind auf der von der Lenkringeinheit abgewandten Seite der Plattform 34 angeordnet, wobei sich die Antriebswelle 17.1 (oder ein entsprechendes Verlängerungsstück) durch eine Öffnung (in Fig. 10 nicht zu sehen) in der Plattform 34 erstreckt, sodass die Antriebsachsen D senkrecht zur gemeinsamen Drehachse A der Antriebsräder 18, 19 und ferner senkrecht zur Längsachse B verlaufen. Zur Sicherung der Längsführung der Zahnstange 24 ist ferner ein Führungsblock 33 benachbart zu der Führungsnut 34.1 auf der Plattform 34 angeordnet und mit Befestigungselementen 33.2 befestigt. Der Führungsblock 33 weist an der der Zahnstange 24 zugewandten Seite eine seitliche Führungsnut 33.1 auf, in der eine Führungsschiene 24.3 der Zahnstange 24 geführt wird, die an der von der Seitenzahnung 24.2 abgewandten Seite der Zahnstange 24 vorliegt.

Fig. 11 verdeutlicht eine Getriebevorrichtung mit einem Antriebskegelrad 26 zur Übertragung der Stellbewegungen vom Motor 17 über die Antriebswelle 17.1 auf das jeweilige Antriebsrad 18, 19, das dazu als Doppelrad auf der nach außen weisenden Seite einen zweiten Kegelradkranz 18.2, 19.2 aufweist, sodass die Antriebsachsen D beider Antriebswellen 17.1 senkrecht zu der gemeinsamen Drehachse A der zwei Antriebsräder 18, 19 verlaufen. Um die Antriebsachsen D möglichst nahe zusammen und kompakt um die Längsachse B anordnen zu können, kann das Doppelrad 18, 19 mit den beiden Kegelradkränzen 18.1, 18.2, 19.1, 19.2 einstückig gefertigt sein, wobei die Anzahl der Zähne des Kegelradkranzes 18.1, 19.1 zum Eingriff mit dem dritten Zahnrad 25 mit der Anzahl der Zähne des zweiten Kegelradkranzes 18.2, 19.2 identisch sein und die Zähne um eine halbe Teilung zueinander versetzt sein können, sodass die Zähne quasi ineinander geschoben sind und die Dicke des Doppelrads minimiert werden kann. Die Antriebsräder 18, 19 können über ein Lager 40 fest in einem nicht dargestellten Gehäuse gelagert sein, sodass ihre gemeinsame Drehachse A und axiale Position definiert sind. Dazu weisen die Antriebsräder 18, 19 einen Achsstummel 18.3, 19.3 auf, der mit dem Lager 40 in einem Gehäuse gelagert werden kann. Alternativ dazu kann die Lagerung umgekehrt einen Achsstummel am Gehäuse vorsehen, auf dem ein in das Antriebsrad 18, 19 eingelassenes Lager sitzt (nicht dargestellt).

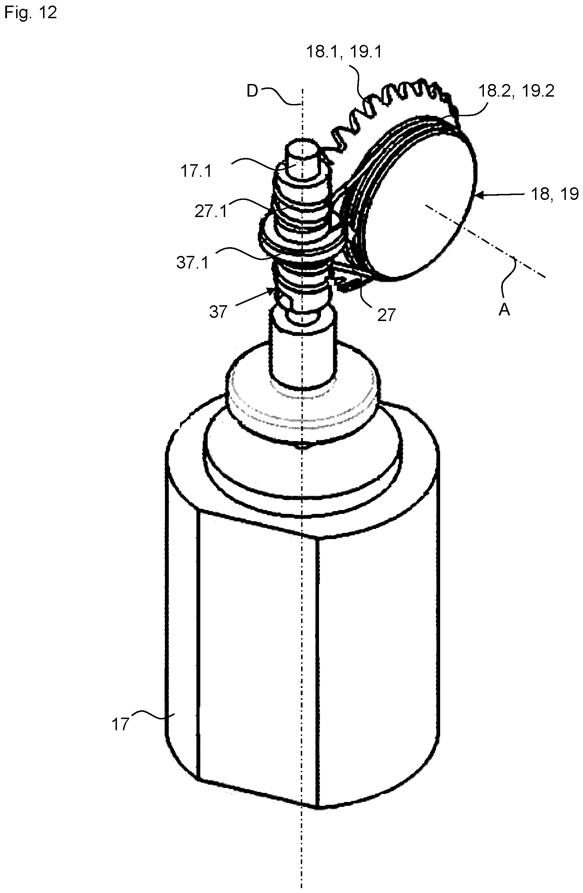

In Fig. 12 ist der Einfachheit halber nur eine Antriebseinheit aus Motor 17, Antriebswelle 17.1 und Getriebevorrichtung für ein Antriebsrad 18,19 dargestellt. Der Aufbau eines damit ausgestatteten erfindungsgemäßen Lenkgetriebes 13 mit einer zweiten derartigen Antriebseinheit, dem dritten Zahnrad 25, dem Lenkring 30 und der Eingriffssicherungsvorrichtung ist aus den übrigen Beispielen ohne weiteres übertragbar. Die Getriebevorrichtung hier weist eine Spindel 37 an der Antriebsachse 17.1 und ein Zugmittel 27 auf, das eine Wirkverbindung zwischen der Spindel 37 und dem hier als Zugmittelscheibe ausgebildeten Antriebskranz 18.2, 19.2 des Antriebsrads 18,19 bereitstellt, das hier als Doppelrad ausgebildet ist, wobei der Kegelradkranz 18.1, 19.1 axial benachbart zu dem Antriebskranz 18.2, 19.2 ausgebildet ist. Unter Antriebskranz 18.2, 19.2 wird in der Ausbildung als Zugmittelscheibe in diesem Zusammenhang eine Scheibe mit zumindest einer umfänglichen Nut zur Führung des Zugmittels 27 verstanden. Auch die Spindel 37 kann eine Nut zur Führung des Zugmittels 27 aufweisen, sodass das Zugmittel 27, das ein Seil oder Riemen sein kann, mit mehreren Zugmittelumschlingungen 27.1 die Spindel 37 umschlingt. Durch die Betätigung der Spindel 37 wird an einem Ende ein Aufwickeln und am anderen Ende ein Abwickeln des Zugmittels 27 auf der Spindel 37 bewirkt, wodurch das Zugmittel 27 die Antriebsbewegung über den als Zugmittelscheibe ausgeführten Antriebskranz 18.2, 19.2 auf das Antriebsrad 18, 19 überträgt.

Das Zugmittel 27 kann durch ein geschlossenes Seil oder einen geschlossenen Riemen bereitgestellt werden, der die Spindel 37 und den Antriebskranz 18.2, 19.2 umschlingt. Alternativ kann, wie in dem Beispiel in Fig. 12 gezeigt, als Zugmittel 27 ein offenes Seil bzw. Riemen mit zwei Enden verwendet werden, die jeweils an den Enden der Spindelnut, die durch eine Spindelmutter 37.1 begrenzt wird, durch Nutensteine (nicht zu sehen) befestigt sind, während das Zugmittel 27 den als Zugmittelscheibe ausgebildeten Antriebskranz 18.2, 19.2 umschlingt. Selbstverständlich kann alternativ die Spindel durch das Zugmittel umschlungen werden, während die Zugmittelenden durch Nutensteine in der Nut des als Zugmittelscheibe ausgebildeten Antriebskranzes 18.2, 19.2 befestigt sind. Ferner besteht auch die Möglichkeit, zwei Zugmittel einzusetzen, wobei jeweils ein Zugmittelende in der Spindelnut und das andere in der Zugmittelscheibennut durch Nutensteine befestigt werden.

In dem in Fig. 12 dargestellten Beispiel verläuft die Antriebsachse D senkrecht zu der gemeinsamen Drehachse A der Antriebsräder 18, 19. Die Position der Motoren 17 ist allerdings durch die Möglichkeit der Zugmittelumlenkung durch ggf. zusätzlichen Einsatz von Umlenkrollen (nicht dargestellt) frei wählbar, sodass die Antriebsachse D in beliebiger Orientierung in Bezug zu der gemeinsamen Drehachse A verlaufen kann.

Fig. 13 verdeutlicht ein Beispiel eines Riemengetriebes als Getriebevorrichtung, das Merkmale des Stirnradgetriebes aus Fig.8 und des Spindelgetriebes aus Fig. 12 kombiniert. Dabei sind die Motoren aus Gründen der besseren Übersichtlichkeit nicht dargestellt, es versteht sich aber von selbst, dass die dargestellte, einem Ritzel entsprechende Riemenscheibe 36 von einem Motor mit einer Antriebswelle, die auf einer Antriebsachse D liegt, angetrieben wird. Infolge der Verwendung von Zugmitteln 27 zur Übertragung der Stellbewegungen auf die Antriebsräder 18, 19 sind die Antriebskränze 18.2, 19.2 der Antriebsräder 18, 19 ebenfalls als Riemenscheiben ausgeführt. Auf diese Weise kann eine Stellbewegung der vom Motor betätigten Riemenscheibe 36 über die Zugmittel 27 auf die Antriebsräder 18, 19 übertragen werden. Wie ferner in Fig. 13 am Antriebskranz 18.2 erkennbar, der eine Zahnung aufweist, ist in diesem Beispiel der Riemen 27 als Zahnriemen ausgeführt. Folglich weist auch die Riemenscheibe 36 eine Zahnung auf, auch wenn diese in der Darstellung nicht ersichtlich ist.

Selbstverständlich sind auch von einem Zahnriemengetriebe abweichende Riemengetriebe, z. B. Flachriemen, Rundriemen, Keilriemen oder Keilrippenriemen, einsetzbar, wobei die Riemenscheiben mit einem dem Antriebsriemen entsprechenden Umfangsprofil ausgebildet sind. Analog zu dem in Fig. 13 gezeigten Beispiel mit Riemengetriebe ist auch eine Ausführungsform mit einem Kettengetriebe denkbar (nicht figurativ dargestellt), bei dem als Zugmittel 27 Ketten eingesetzt werden und entsprechend die Riemenscheibe 36 sowie die Antriebskränze 18.2, 19.2 der Antriebsräder 18, 19 als Kettenräder ausgeführt sind. In beiden Fällen ist die Positionierung der Riemenscheiben 36 bzw. der Antriebsachsen D nicht nur unabhängig voneinander und beliebig auf einer Kreisbahn um die Antriebsräder 18, 19, sondern durch Variation der Länge des Zugmittels auch in beliebigem Abstand von den Antriebsrädern 18, 19 gewählt werden kann. Da jedoch eine Bauraumreduktion erstrebenswert ist, ist eine Positionierung der Antriebsachsen D nahe der Antriebsräder 18, 19, und vorzugsweise auch nahe der Längsachse B bevorzugt.

Die im Zusammenhang mit den Figuren beschriebenen Beispiele sollen nicht als beschränkend verstanden werden. Beschriebene Details wie die Ausführungsvarianten der Antriebsräder 18, 19 als Doppelrad oder mit unterschiedlichen Zahnungsabschnitten, oder die Einstückigkeit von Lenkring 30 und drittem Zahnrad 25 können in beliebiger Weise, sofern sinnvoll, kombiniert werden.

In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen. Die vorliegende Erfindung stellt ein Lenkgetriebe 13 für ein chirurgisches Instrument 1 bereit, wobei das Lenkgetriebe 13 am proximalen Ende 3 eines Schafts 2 anordenbar ist, der eine Längsachse B definiert und am distalen Ende 5 eine Abwinkelungsmechanik 9 aufweist, die durch eine räumlich ausrichtbare Taumelscheibe 14 steuerbar ist. Dabei weist das Lenkgetriebe 13 ein erstes Antriebsrad 18 und ein zweites Antriebsrad 19 mit jeweils einem zugeordneten Motor 17 auf, und das zweite Antriebsrad 19 ist um 180 ° zum ersten Antriebsrad 18 versetzt auf einer mit dem ersten Antriebsrad 18 gemeinsamen, senkrecht zur Längsachse B verlaufenden Drehachse A angeordnet. Die Taumelscheibe 14 ist zwischen dem ersten Antriebsrad 18 und dem zweiten Antriebsrad 19 in einem Lenkring 30 gelagert, der drehfest mit einem dritten Zahnrad 25 verbunden ist, das mit dem ersten Antriebsrad 18 und dem zweiten Antriebsrad 19 in Eingriff steht und um eine Drehachse C drehbar ist, die senkrecht zu der gemeinsamen Drehachse A der Antriebsräder 18, 19 verläuft. Dabei weist das Lenkgetriebe 13 einen Haltebügel 20 auf, der drehbar um die gemeinsame Drehachse A der Antriebsräder 18, 19 gelagert ist, wobei der Lenkring 30 in dem Haltebügel 20 drehbar um die Drehachse C des dritten Zahnrads 25 gelagert ist, sodass der Eingriff des dritten Zahnrads 25 mit den Antriebsrädern 18, 19 gesichert ist. Ferner wird ein chirurgisches Instrument 1 offenbart, das ein solches Lenkgetriebe 13 aufweist.

### BEZUGSZEICHENLISTE

- 1: Chirurgisches Instrument
- 2: Schaft
- 3: Proximales Ende (Schaft)
- 4: Betätigungseinheit
- 5: Distales Ende (Schaft)
- 6: Werkzeugspitze
- 7: Werkzeug
- 8: Betätigungselement
- 9: Gelenkmechanismus
- 11: Schwenkglied
- 12: Lenkdraht
- 13: Lenkgetriebe
- 14: Taumelscheibe (räumlich verstellbar)
- 15, 16: Ritzel
- 17: Motor
- 17.1: Antriebswelle
- 18: Antriebsrad
- 18.1, 18.2: Kegelradkranz(abschnitt), Antriebskranz(abschnitt)
- 18.3, 18.4: Achsstummel, Abtriebswelle
- 19: Antriebsrad
- 19.1, 19.2: Kegelradkranz(abschnitt), Antriebskranz(abschnitt)
- 19.3: Achsstummel
- 20: Haltebügel
- 20.1: Aufnahmeöffnung
- 20.2, 20.3: Schenkel, Basis
- 20.4, 20.5: Lagerauge, Lagersitz
- 21: Hauptwelle
- 22: Fächerscheibe
- 23: Schneckenwelle
- 24: Zahnstange
- 24.1: Obere Zahnung
- 24.2: Seitliche Zahnung
- 24.3: Führungsschiene
- 24.4: Führungsabschnitt
- 25, 25.1: Drittes Zahnrad, Kegelradkranz(abschnitt)
- 26: Kegelrad
- 27, 27.1: Zugmittel, Umschlingung
- 28: Befestigungselement,
- 28.1: Schraubkopf
- 29: Lagerring
- 30: Lenkring
- 30.1: Lenkringstutzen,
- 31, 31.1: Viertes Zahnrad, Kegelradkranz
- 32: Bügellager
- 33: Führungsblock
- 33.1: Führungsnut
- 33.2: Befestigungselement
- 34: Plattform
- 34.1: Führungsnut
- 34.2: Lagerbügel
- 36: Riemenscheibe
- 37: Spindel
- 37.1: Spindelmutter
- 40: Lager (im Gehäuse)
- 42: Lenkringlager
- 43: Halterung
- A: Gemeinsame Drehachse Antriebsräder
- B: Längsachse
- C: Drehachse drittes Zahnrad
- D: Antriebsachse

## Patentansprüche

1. Lenkgetriebe (13) für ein chirurgisches Instrument (1), wobei das Lenkgetriebe (13) eine räumlich ausrichtbare Taumelscheibe (14) aufweist, wobei das Lenkgetriebe (13) am proximalen Ende (3) eines Schafts (2) anordenbar ist, der eine Längsachse (B) definiert und am distalen Ende (5) eine Abwinkelungsmechanik (9) aufweist, die durch die Taumelscheibe (14) steuerbar ist, wobei
- das Lenkgetriebe (13) ein erstes Antriebsrad (18) und ein zweites Antriebsrad (19) mit jeweils einem zugeordneten Motor (17) aufweist, und
- das zweite Antriebsrad (19) um 180 ° zum ersten Antriebsrad (18) versetzt auf einer mit dem ersten Antriebsrad (18) gemeinsamen, senkrecht zur Längsachse (B) verlaufenden Drehachse (A) angeordnet ist, und
- die Taumelscheibe (14) zwischen dem ersten Antriebsrad (18) und dem zweiten Antriebsrad (19) in einem Lenkring (30) gelagert ist, der drehfest mit einem dritten Zahnrad (25) verbunden ist, das mit dem ersten Antriebsrad (18) und dem zweiten Antriebsrad (19) in Eingriff steht und um eine Drehachse (C) drehbar ist, die senkrecht zu der gemeinsamen Drehachse (A) der Antriebsräder (18, 19) verläuft, **dadurch gekennzeichnet, dass**
das Lenkgetriebe (13) einen Haltebügel (20) aufweist, der drehbar um die gemeinsame Drehachse (A) der Antriebsräder (18, 19) gelagert ist, wobei der Lenkring (30) in dem Haltebügel (20) drehbar um die Drehachse (C) des dritten Zahnrads (25) gelagert ist, sodass der Eingriff des dritten Zahnrads (25) mit den Antriebsrädern (18, 19) gesichert ist.

2. Lenkgetriebe (13) nach Anspruch 1,
**dadurch gekennzeichnet, dass**
der Haltebügel (20) u-förmig mit einer Basis (20.3) und zwei Schenkeln (20.2) ausgebildet ist, wobei die Basis (20.3) eine Aufnahmeöffnung (20.1) aufweist, in der ein Lenkringstutzen (30.1), der an dem Lenkring (30) ausgebildet ist, drehbar gelagert ist, und wobei jeder Schenkel (20.2) ein Lagerauge (20.4) aufweist, mit dem der Haltebügel (20) drehbar um die gemeinsame Drehachse (A) der Antriebsräder (18, 19), bevorzugt drehbar auf einem Achsstummel (18.3, 19.3) der Antriebsräder (18, 19), gelagert ist.

3. Lenkgetriebe (13) nach Anspruch 2,
**dadurch gekennzeichnet, dass**
der Lenkringstutzen (30.1) zylindrisch geformt ist und über ein Lenkringlager (42) in einem zylindrischen Abschnitt der Aufnahmeöffnung (20.1) gelagert ist.

4. Lenkgetriebe (13) nach Anspruch 2 oder 3,
**dadurch gekennzeichnet, dass**
das dritte Zahnrad (25) an dem Lenkring (30) diametral zur Basis (20.3) des Haltebügels (20) vorliegt, oder
bevorzugt das dritte Zahnrad (25) und die Basis (20.3) des Haltebügels (20) auf der gleichen Seite des Lenkrings (30) vorliegen.

5. Lenkgetriebe (13) nach Anspruch 4,
**dadurch gekennzeichnet, dass**
das Lenkgetriebe (13), bei dem das dritte Zahnrad (25) an dem Lenkring (30) diametral zur Basis (20.3) des Haltebügels (20) vorliegt, ein Befestigungselement (28) aufweist, das eine Wirkverbindung zwischen dem Haltebügel (20) und dem Lenkring (30) bereitstellt und dazu ausgebildet ist, den Lenkring (30) mit dem verbundenen dritten Zahnrad (25) in Richtung der Basis (20.3) des Haltebügels (20) zu ziehen, um den Eingriff des Zahnrads (25) mit den Antriebsrädern (18, 19) zu sichern.

6. Lenkgetriebe (13) nach Anspruch 5,
**dadurch gekennzeichnet, dass**
das Befestigungselement (28) des Lenkgetriebes (13) mit dem zylindrisch geformten Lenkringstutzen (30.1), der über das Lenkringlager (42) in dem zylindrischen Abschnitt der Aufnahmeöffnung (20.1) gelagert ist, eine Schraube (28) ist, die in den Lenkringstutzen (30.1) in der Drehachse (C) des dritten Zahnrads (25) eingezogen ist.

7. Lenkgetriebe (13) nach zumindest einem der Ansprüche 4 bis 6,
**dadurch gekennzeichnet, dass**
- der Lenkring (30) einstückig mit dem dritten Zahnrad (25) ausgebildet ist, und / oder
- die Antriebsräder (18, 19) jeweils zumindest einen Kegelradkranzabschnitt (18.1, 19.1) zum Eingriff mit zumindest einem Kegelradkranzabschnitt (25.1) des dritten Zahnrads (15) aufweisen, wobei die Kegelradkranzabschnitte (18.1, 19.1) der Antriebsräder (18, 19) und der Kegelradkranzabschnitt (25.1) des dritten Zahnrads (15) vollumfänglich als Kegelkränze (18.1, 19.1, 25.1) ausgebildet sind oder sich über einen ersten vorbestimmbaren umfänglichen Abschnitt des jeweiligen Antriebsrads (18, 19) und über vorbestimmbare umfängliche Abschnitte des dritten Zahnrad (25) erstrecken, die den Eingriff der Antriebsräder (18, 19) mit dem dritten Zahnrad (25) in einem vorbestimmbaren Bewegungsumfang des Lenkrings (30) bereitstellen.

8. Lenkgetriebe (13) nach zumindest einem der Ansprüche 1 bis 7,
**dadurch gekennzeichnet, dass**
jedes Antriebsrad (18, 19) von dem zugeordneten Motor (17) über jeweils eine Antriebswelle (17.1) betätigbar ist, deren Antriebsachse der gemeinsamen Drehachse (A) der zwei Antriebsräder (18, 19) entspricht,
oder
jedes Antriebsrad (18, 19) von dem zugeordneten Motor (17) über jeweils eine Antriebswelle (17.1) und jeweils eine Getriebevorrichtung betätigbar ist, wobei die Antriebsachse (D) der Antriebswelle (17.1) nicht der gemeinsamen Drehachse (A) der zwei Antriebsräder (18, 19) entspricht.

9. Lenkgetriebe (13) nach Anspruch 8,
**dadurch gekennzeichnet, dass**
die Getriebevorrichtung
- ein Ritzel (15) ist, und die Antriebsräder (18, 19) jeweils zumindest einen Antriebskranzabschnitt (18.2, 19.2) zum Eingriff mit dem jeweiligen Ritzel (15) aufweisen, wobei die Antriebsachsen (D) beider Antriebswellen (17.1) parallel zu der gemeinsamen Drehachse (A) der zwei Antriebsräder (18, 19) verlaufen, oder
- eine Schneckenwelle (23) ist, und die Antriebsräder (18, 19) jeweils zumindest einen Antriebskranzabschnitt (18.2, 19.2) zum Eingriff mit der jeweiligen Schneckenwelle (23) aufweisen, wobei die Antriebsachsen (D) senkrecht zu der gemeinsamen Drehachse (A) der zwei Antriebsräder (18, 19) verlaufen, oder
- ein Antriebskegelrad (26) ist, und die Antriebsräder (18, 19) jeweils zumindest einen Antriebskranzabschnitt (18.2, 19.2) zum Eingriff mit dem jeweiligen Antriebskegelrad (26) aufweisen, wobei die Antriebsachsen (D) beider Antriebswellen (17.1) senkrecht zu der gemeinsamen Drehachse (A) der zwei Antriebsräder (18, 19) verlaufen.

10. Lenkgetriebe (13) nach Anspruch 9,
**dadurch gekennzeichnet, dass**
- die Antriebsräder (18, 19) als Doppelräder (18, 19) ausgebildet sind, wobei der jeweilige Kegelradkranzabschnitt (18.1, 19.1) axial benachbart zu dem Antriebskranzabschnitt (18.2, 19.2) angeordnet ist und die Kegelradkranzabschnitte (18.1, 19.1) der beiden Doppelräder (18, 19) zum Eingriff mit dem dritten Zahnrad (25) einander zugewandt sind,
oder
- der jeweilige Antriebskranzabschnitt (18.2, 19.2) sich über einen zweiten vorbestimmbaren umfänglichen Abschnitt des jeweiligen Antriebsrads (18, 19) erstrecken, der den Eingriff der Antriebsräder (18, 19) mit dem jeweiligen Ritzel (15) oder der jeweiligen Schneckenwelle (23) oder dem jeweiligen Antriebskegelrad (26) in einem vorbestimmbaren Bewegungsumfang des Lenkrings (30) bereitstellt, wobei der Antriebskranzabschnitt (18.2, 19.2) keine Überschneidung mit dem jeweiligen Kegelradkranzabschnitt (18.1, 19.1) aufweist.

11. Lenkgetriebe (13) nach Anspruch 8,
**dadurch gekennzeichnet, dass**
die Getriebevorrichtung ein Antriebsritzel (15), eine Zahnstange (24), die eine Stirnzahnung (24.1) und eine Seitenzahnung (24.2) aufweist, und ein Übertragungsritzel (16) aufweist, wobei
- das Antriebsritzel (15) über die Antriebswelle (17.1) von dem jeweils zugeordneten Motor (17) betätigbar ist und mit der Seitenzahnung (24.2) der Zahnstange (24) in Eingriff steht, und
- das Übertragungsritzel (16) mit der Stirnzahnung (24.1) der Zahnstange (24) in Eingriff steht und über eine Abtriebswelle (18.4) mit dem Antriebsrad (18, 19) verbunden ist,
wobei die Antriebsachsen (D) senkrecht zu der gemeinsamen Drehachse (A) der zwei Antriebsräder (18, 19) verlaufen.

12. Lenkgetriebe (13) nach Anspruch 8,
**dadurch gekennzeichnet, dass**
die Getriebevorrichtung eine Spindel (37) und ein Zugmittel (27) aufweist, und die Antriebsräder (18, 19) als Doppelräder (18, 19) ausgebildet sind, wobei Antriebskranz (18.2, 19.2) als Zugmittelscheibe ausgebildet ist, und wobei das Zugmittel (27) eine Wirkverbindung zwischen der Spindel (37), die an der Antriebswelle (17.1) angeordnet ist, und dem als Zugmittelscheibe ausgebildeten Antriebskranz (18.2, 19.2) bereitstellt, wobei eine Orientierung der Antriebsachsen (D) in Bezug auf die gemeinsame Drehachse (A) der zwei Antriebsräder (18, 19) beliebig wählbar ist.

13. Lenkgetriebe (13) nach Anspruch 8,
**dadurch gekennzeichnet, dass**
die Getriebevorrichtung eine Riemenscheibe (36) und ein Zugmittel (27) aufweist, und die Antriebsräder (18, 19) als Doppelräder (18, 19) ausgebildet sind, wobei Antriebskranz (18.2, 19.2) als Riemenscheibe ausgebildet ist, und wobei das Zugmittel (27) eine Wirkverbindung zwischen der Riemenscheibe (36), die an der Antriebswelle (17.1) angeordnet ist, und dem als Zugmittelscheibe ausgebildeten Antriebskranz (18.2, 19.2) bereitstellt, wobei die Antriebsachsen (D) parallel zu der gemeinsamen Drehachse (A) der zwei Antriebsräder (18, 19) verlaufen.

14. Chirurgisches Instrument (1), das einen Schaft (2), eine am proximalen Ende (3) des Schaftes (2) angeordnete Betätigungseinheit (4) und ein am distalen Ende (5) des Schaftes (2) angeordnetes Werkzeug (7) mit einer mittels einer distalen Abwinkelungsmechanik (9) abwinkelbaren Werkzeugspitze (6) aufweist, die durch eine mittels zweier Antriebe räumlich ausrichtbare Taumelscheibe (14) steuerbar ist,
**dadurch gekennzeichnet, dass**
das chirurgische Instrument (1) ein Lenkgetriebe (13) nach zumindest einem der Ansprüche 1 bis 13 zur räumlichen Ausrichtung der Taumelscheibe (14) aufweist.

15. Chirurgisches Instrument (1) nach Anspruch 14,
**dadurch gekennzeichnet, dass**
die Taumelscheibe (14) kardanisch mit einer koaxial zu einer Längsachse (B) des Schaftes (2) verlaufenden Hauptwelle (21) gekoppelt ist, und / oder
ein Betätigungselement (8) axial verschiebbar in dem Schaft (2) gelagert ist und proximalseitig mit der Betätigungseinheit (4) in Wirkverbindung steht, und / oder die distalen Abwinkelungsmechanik (9) der abwinkelbaren Werkzeugspitze (6) aus an dem distalen Ende (5) des Schaftes (2) angeordneten Schwenkgliedern (11) besteht, die über in Längsrichtung des Schaftes (2) verlaufende Lenkdrähte (12) mit dem Lenkgetriebe (13) verbunden sind, und wobei bevorzugt distalseitig vor der räumlich verstellbaren Scheibe (14) eine Fächerscheibe (22) auf der Hauptwelle (21) angeordnet ist, die den radialen Abstand der Lenkdrähte (12) von der Längsachse (B) des Schaftes (2) vergrößert.

## Claims

1. A steering gear (13) for a surgical instrument (1), wherein the steering gear (13) has a spatially alignable swash plate (14), wherein the steering gear (13) can be arranged at the proximal end (3) of a shaft (2) which defines a longitudinal axis (B) and has, at the distal end (5), a bending mechanism (9), which can be controlled by the swash plate (14), wherein
- the steering gear (13) has a first drive wheel (18) and a second drive wheel (19), each with an assigned motor (17), and
- the second drive wheel (19) is arranged offset by 180° relative to the first drive wheel (18) on an axis of rotation (A) common to the first drive wheel (18) and running perpendicular to the longitudinal axis (B), and
- the swash plate (14) is mounted between the first drive wheel (18) and the second drive wheel (19) in a steering ring (30) which is connected in a rotationally fixed manner to a third gear wheel (25) which engages with the first drive wheel (18) and the second drive wheel (19) and is rotatable about an axis of rotation (C) which runs perpendicular to the common axis of rotation (A) of the drive wheels (18, 19), **characterised in that**
the steering gear (13) has a retaining bracket (20) which is rotatably mounted about the common axis of rotation (A) of the drive wheels (18, 19), wherein the steering ring (30) is mounted in the retaining bracket (20) rotatably about the axis of rotation (C) of the third gear wheel (25) such that the engagement of the third gear wheel (25) with the drive wheels (18, 19) is secured.

2. The steering gear (13) according to claim 1,
**characterised in that**
the retaining bracket (20) is U-shaped with a base (20.3) and two limbs (20.2), wherein the base (20.3) has a receptacle opening (20.1) in which a steering ring connector (30.1), which is formed on the steering ring (30), is rotatably mounted, and wherein each limb (20.2) has a bearing eye (20.4), with which the retaining bracket (20) is rotatably mounted about the common axis of rotation (A) of the drive wheels (18, 19), preferably rotatably on an axis stub (18.3, 19.3) of the drive wheels (18, 19).

3. The steering gear (13) according to claim 2,
**characterised in that**
the steering ring connector (30.1) is cylindrical in shape and is mounted in a cylindrical section of the receptacle opening (20.1) via a steering ring bearing (42).

4. The steering gear (13) according to claim 2 or 3,
**characterised in that**
the third gear wheel (25) is present on the steering ring (30) diametrically to the base (20.3) of the retaining bracket (20), or
preferably the third gear wheel (25) and the base (20.3) of the retaining bracket (20) are on the same side of the steering ring (30).

5. The steering gear (13) according to claim 4,
**characterised in that**
the steering gear (13), in which the third gear wheel (25) is present on the steering ring (30) diametrically to the base (20.3) of the retaining bracket (20), has a fastening element (28) which provides an operative connection between the retaining bracket (20) and the steering ring (30) and is designed to pull the steering ring (30) with the connected third gear wheel (25) in the direction of the base (20.3) of the retaining bracket (20) in order to secure the engagement of the gear wheel (25) with the drive wheels (18, 19).

6. The steering gear (13) according to claim 5,
**characterised in that**
the fastening element (28) of the steering gear (13) with the cylindrically shaped steering ring connector (30.1), which is mounted via the steering ring bearing (42) in the cylindrical section of the receptacle opening (20.1), is a screw (28) which is screwed into the steering ring connector (30.1) in the axis of rotation (C) of the third gear wheel (25).

7. The steering gear (13) according to at least one of claims 4 to 6, **characterised in that**
- the steering ring (30) is formed in one piece with the third gear wheel (25), and/or
- the drive wheels (18, 19) each have at least one bevel gear rim section (18.1, 19.1) for engagement with at least one bevel gear rim section (25.1) of the third gear wheel (15), wherein the bevel gear rim sections (18.1, 19.1) of the drive wheels (18, 19) and the bevel gear rim section (25.1) of the third gear wheel (15) are formed entirely as bevel rims (18.1, 19.1, 25.1) or extend over a first predeterminable circumferential section of the respective drive wheel (18, 19) and over predeterminable circumferential sections of the third gear wheel (25), which provide the engagement of the drive wheels (18, 19) with the third gear wheel (25) in a predeterminable range of motion of the steering ring (30).

8. The steering gear (13) according to at least one of claims 1 to 7,
**characterised in that**
each drive wheel (18, 19) can be actuated by the assigned motor (17) via a drive shaft (17.1) in each case, the drive axis of which corresponds to the common axis of rotation (A) of the two drive wheels (18, 19),
or
each drive wheel (18, 19) can be actuated by the assigned motor (17) via a drive shaft (17.1) in each case and a gear mechanism in each case, wherein the drive axis (D) of the drive shaft (17.1) does not correspond to the common axis of rotation (A) of the two drive wheels (18, 19).

9. The steering gear (13) according to claim 8,
**characterised in that**
the gear mechanism
- is a pinion (15), and the drive wheels (18, 19) each have at least one drive rim section (18.2, 19.2) for engagement with the respective pinion (15), wherein the drive axes (D) of both drive shafts (17.1) run parallel to the common axis of rotation (A) of the two drive wheels (18, 19), or
- is a worm shaft (23), and the drive wheels (18, 19) each have at least one drive rim section (18.2, 19.2) for engagement with the respective worm shaft (23), wherein the drive axes (D) run perpendicular to the common axis of rotation (A) of the two drive wheels (18, 19), or
- is a drive bevel gear (26), and the drive wheels (18, 19) each have at least one drive rim section (18.2, 19.2) for engagement with the respective drive bevel gear (26), wherein the drive axes (D) of both drive shafts (17.1) run perpendicular to the common axis of rotation (A) of the two drive wheels (18, 19).

10. The steering gear (13) according to claim 9,
**characterised in that**
- the drive wheels (18, 19) are designed as double wheels (18, 19), wherein the respective bevel gear rim section (18.1, 19.1) is arranged axially adjacent to the drive rim section (18.2, 19.2) and the bevel gear rim sections (18.1, 19.1) of the two double wheels (18, 19) face each other for engagement with the third gear wheel (25),
or
- the respective drive rim section (18.2, 19.2) extends over a second predeterminable circumferential section of the respective drive wheel (18, 19) which provides the engagement of the drive wheels (18, 19) with the respective pinion (15) or the respective worm shaft (23) or the respective drive bevel gear (26) in a predeterminable range of motion of the steering ring (30), wherein the drive rim section (18.2, 19.2) has no overlap with the respective bevel gear rim section (18.1, 19.1).

11. The steering gear (13) according to claim 8,
**characterised in that**
the gear mechanism has a drive pinion (15), a toothed rack (24), which has an end toothing (24.1) and a lateral toothing (24.2), and a transmission pinion (16), wherein
- the drive pinion (15) can be actuated by the respectively assigned motor (17) via the drive shaft (17.1) and engages with the lateral toothing (24.2) of the toothed rack (24), and
- the transmission pinion (16) engages with the end toothing (24.1) of the toothed rack (24) and is connected to the drive wheel (18, 19) via an output shaft (18.4),
wherein the drive axes (D) run perpendicular to the common axis of rotation (A) of the two drive wheels (18, 19).

12. The steering gear (13) according to claim 8,
**characterised in that**
the gear mechanism has a spindle (37) and a traction means (27), and the drive wheels (18, 19) are designed as double wheels (18, 19), wherein the drive rim (18.2, 19.2) is designed as a traction means plate, and wherein the traction means (27) provides an operative connection between the spindle (37), which is arranged on the drive shaft (17.1), and the drive rim (18.2, 19.2) designed as a traction means plate, wherein an orientation of the drive axes (D) in relation to the common axis of rotation (A) of the two drive wheels (18, 19) can be selected as desired.

13. The steering gear (13) according to claim 8,
**characterised in that**
the gear mechanism has a belt pulley (36) and a traction means (27), and the drive wheels (18, 19) are designed as double wheels (18, 19), wherein the drive rim (18.2, 19.2) is designed as a belt pulley, and wherein the traction means (27) provides an operative connection between the belt pulley (36), which is arranged on the drive shaft (17.1), and the drive rim (18.2, 19.2) designed as a traction means plate, wherein the drive axes (D) run parallel to the common axis of rotation (A) of the two drive wheels (18, 19).

14. A surgical instrument (1) having a shaft (2), an actuation unit (4) arranged at the proximal end (3) of the shaft (2) and a tool (7) arranged at the distal end (5) of the shaft (2) with a tool tip (6) which can be angled by means of a distal bending mechanism (9) and which can be controlled by a swash plate (14) which can be spatially aligned by means of two drives, **characterised in that**
the surgical instrument (1) has a steering gear (13) according to at least one of claims 1 to 13 for the spatial orientation of the swash plate (14).

15. The surgical instrument (1) according to claim 14,
**characterised in that**
the swash plate (14) is cardanically coupled to a main shaft (21) running coaxially to a longitudinal axis (B) of the shaft (2), and/or
an actuating element (8) is mounted so as to be axially displaceable in the shaft (2) and is operatively connected to the actuation unit (4) on the proximal side, and/or the distal bending mechanism (9) of the bendable tool tip (6) consists of swivel members (11) which are arranged at the distal end (5) of the shaft (2) and which are connected to the steering gear (13) via steering wires (12) running in the longitudinal direction of the shaft (2), and wherein a serrated lock washer (22) is preferably arranged on the main shaft (21) on the distal side in front of the spatially adjustable plate (14) and increases the radial distance of the steering wires (12) from the longitudinal axis (B) of the shaft (2).

## Revendications

1. Appareil de direction (13) pour un instrument chirurgical (1), dans lequel l'appareil de direction (13) a un plateau oscillant (14) pouvant être aligné dans l'espace, dans lequel l'appareil de direction (13) peut être disposé à l'extrémité proximale (3) d'un arbre (2) qui définit un axe longitudinal (B) et a, à l'extrémité distale (5), un mécanisme de flexion (9), qui peut être contrôlé par le plateau oscillant (14), dans lequel
- l'appareil de direction (13) a une première roue motrice (18) et une deuxième roue motrice (19), chacune avec un moteur assigné (17), et
- la deuxième roue motrice (19) est décalée de 180° par rapport à la première roue motrice (18) sur un axe de rotation (A) commun à la première roue motrice (18) et s'étendant perpendiculaire à l'axe longitudinal (B), et
- le plateau oscillant (14) est monté entre la première roue motrice (18) et la deuxième roue motrice (19) dans un anneau de direction (30) qui est relié de manière fixe en rotation à une troisième roue dentée (25) qui est en prise avec la première roue motrice (18) et la deuxième roue motrice (19) et qui peut tourner autour d'un axe de rotation (C) qui s'étend perpendiculaire à l'axe de rotation commun (A) des roues d'entraînement (18, 19), **caractérisé en ce que**
l'appareil de direction (13) a un support de retenue (20) qui est monté rotatif autour de l'axe de rotation commun (A) des roues d'entraînement (18, 19), dans lequel l'anneau de direction (30) est monté dans le support de retenue (20) de manière rotative autour de l'axe de rotation (C) de la troisième roue dentée (25), de sorte que la mise en prise de la troisième roue dentée (25) avec les roues d'entraînement (18, 19) est assuré.

2. Appareil de direction (13) selon la revendication 1,
**caractérisé en ce que**
le support de retenue (20) est en forme de U avec une base (20.3) et deux branches (20.2), dans lequel la base (20.3) a une ouverture réceptrice (20.1) dans laquelle un connecteur d'anneau de direction (30.1), qui est formé sur l'anneau de direction (30), est monté de manière rotatif, et dans lequel chaque branche (20.2) a un d'œillet de palier (20.4), avec lequel le support de retenue (20) est monté de manière rotatif autour de l'axe de rotation commun (A) des roues d'entraînement (18, 19), de préférence de manière rotatif sur un embout d'axe (18.3, 19.3) des roues d'entraînement (18, 19).

3. Appareil de direction (13) selon la revendication 2,
**caractérisé en ce que**
le connecteur d'anneau de direction (30.1) est de forme cylindrique et est monté dans une section cylindrique de l'ouverture réceptrice (20.1) par l'intermédiaire d'un palier d'anneau de direction (42).

4. Appareil de direction (13) selon la revendication 2 ou 3,
**caractérisé en ce que**
la troisième roue dentée (25) est présente sur l'anneau de direction (30) diamétralement à la base (20.3) du support de retenue (20), ou
de préférence, la troisième roue dentée (25) et la base (20.3) du support de retenue (20) se trouvent du même côté de l'anneau de direction (30).

5. Appareil de direction (13) selon la revendication 4,
**caractérisé en ce que**
l'appareil de direction (13), dans lequel la troisième roue dentée (25) est présente sur l'anneau de direction (30) diamétralement à la base (20.3) du support de retenue (20), a un élément de fixation (28) qui fournit une liaison opérationnelle entre le support de retenue (20) et l'anneau de direction (30) et qui est conçu pour tirer l'anneau de direction (30) avec la troisième roue dentée (25) connectée en direction de la base (20.3) du support de retenue (20) afin d'assurer la mise en prise de la roue dentée (25) avec les roues d'entraînement (18, 19).

6. Appareil de direction (13) selon la revendication 5,
**caractérisé en ce que**
l'élément de fixation (28) de l'appareil de direction (13) avec le connecteur d'anneau de direction (30.1) de forme cylindrique, qui est monté par l'intermédiaire du palier d'anneau de direction (42) dans la section cylindrique de l'ouverture réceptrice (20.1), est une vis (28) qui est vissée dans le connecteur d'anneau de direction (30.1) dans l'axe de rotation (C) de la troisième roue dentée (25).

7. Appareil de direction (13) selon au moins l'une des revendications 4 à 6, **caractérisé en ce que**
- l'anneau de direction (30) est formé d'une seule pièce avec la troisième roue dentée (25), et/ou
- les roues d'entraînement (18, 19) ont chacune au moins une section de couronne conique (18.1, 19.1) pour la mise en prise avec au moins une section de couronne conique (25.1) de la troisième roue dentée (15), dans lequel les sections de couronne conique (18.1, 19.1) des roues d'entraînement (18, 19) et la section de la couronne conique (25.1) de la troisième roue dentée (15) sont entièrement formées de bords coniques (18.1, 19.1, 25.1) ou s'étendent sur une première section circonférentielle prédéterminée de la roue motrice respective (18, 19) et sur des sections circonférentielles prédéterminées de la troisième roue dentée (25), qui assurent la mise en prise des roues d'entraînement (18, 19) avec la troisième roue dentée (25) dans une amplitude de mouvement prédéterminée de l'anneau de direction (30).

8. Appareil de direction (13) selon au moins l'une des revendications 1 à 7, **caractérisé en ce que**
chaque roue motrice (18, 19) peut être actionnée par le moteur assigné (17) par l'intermédiaire d'un arbre d'entraînement (17.1) dans chaque cas, dont l'axe d'entraînement correspond à l'axe de rotation commun (A) des deux roues d'entraînement (18, 19),
ou
chaque roue motrice (18, 19) peut être actionnée par le moteur assigné (17) par l'intermédiaire d'un arbre d'entraînement (17.1) dans chaque cas et d'un mécanisme d'engrenage dans chaque cas, dans lequel l'axe d'entraînement (D) de l'arbre d'entraînement (17.1) ne correspond pas à l'axe de rotation commun (A) des deux roues d'entraînement (18, 19).

9. Appareil de direction (13) selon la revendication 8,
**caractérisé en ce que**
le mécanisme d'engrenage
- est un pignon (15), et les roues d'entraînement (18, 19) ont chacune au moins une section de bord d'entraînement (18.2, 19.2) pour la mise en prise avec le pignon respectif (15), dans lequel les axes d'entraînement (D) des deux arbres d'entraînement (17.1) s'étendent parallèles à l'axe de rotation commun (A) des deux roues d'entraînement (18, 19), ou
- est un arbre à vis sans fin (23), et les roues d'entraînement (18, 19) ont chacune au moins une section de bord d'entraînement (18.2, 19.2) pour la mise en prise avec l'arbre à vis sans fin respectif (23), dans lequel les axes d'entraînement (D) s'étendent perpendiculaires à l'axe de rotation commun (A) des deux roues d'entraînement (18, 19), ou
- est un engrenage conique d'entraînement (26), et les roues d'entraînement (18, 19) ont chacune au moins une section de bord d'entraînement (18.2, 19.2) pour la mise en prise avec l'engrenage conique d'entraînement respectif (26), dans lequel les axes d'entraînement (D) des deux arbres d'entraînement (17.1) s'étendent perpendiculaires à l'axe de rotation commun (A) des deux roues d'entraînement (18, 19).

10. Appareil de direction (13) selon la revendication 9,
**caractérisé en ce que**
- les roues d'entraînement (18, 19) sont conçues comme des roues doubles (18, 19), dans lequel la section de couronne conique respectif (18.1, 19.1) est disposée axialement adjacente à la section de bord d'entraînement (18.2, 19.2) et les sections de couronne conique (18.1, 19.1) des deux roues doubles (18, 19) se font face pour la mise en prise avec la troisième roue dentée (25),
ou
- la section de bord d'entraînement respective (18.2, 19.2) s'étend sur une deuxième section circonférentielle prédéterminable de la roue d'entraînement respective (18, 19) qui fournit la mise en prise des roues d'entraînement (18, 19) avec le pignon respectif (15) ou l'arbre à vis sans fin respectif (23) ou l'engrenage conique d'entraînement respectif (26) dans une amplitude de mouvement prédéterminée de l'anneau de direction (30), dans lequel la section de bord d'entraînement (18.2, 19.2) ne se chevauche pas avec la section de couronne conique respectif (18.1, 19.1).

11. Appareil de direction (13) selon la revendication 8,
**caractérisé en ce que**
le mécanisme d'engrenage a un pignon d'entraînement (15), une crémaillère (24), qui a une denture d'extrémité (24.1) et une denture latérale (24.2), et un pignon de transmission (16), dans lequel
- le pignon d'entraînement (15) peut être actionné par le moteur assigné respectif (17) par l'intermédiaire de l'arbre d'entraînement (17.1) et est en prise avec la denture latérale (24.2) de la crémaillère (24), et
- le pignon de transmission (16) est en prise avec la denture d'extrémité (24.1) de la crémaillère (24) et est relié à la roue motrice (18, 19) par l'intermédiaire d'un arbre de sortie (18.4),
dans lequel les axes d'entraînement (D) s'étendent perpendiculaires à l'axe de rotation commun (A) des deux roues d'entraînement (18, 19).

12. Appareil de direction (13) selon la revendication 8,
**caractérisé en ce que**
le mécanisme d'engrenage a une broche (37) et un moyen de traction (27), et les roues d'entraînement (18, 19) sont conçues comme des roues doubles (18, 19), dans lequel le bord d'entraînement (18.2, 19.2) est conçue comme un plateau de moyen de traction, et dans lequel le moyen de traction (27) fournit une liaison opérationnelle entre la broche (37), qui est disposée sur l'arbre d'entraînement (17.1), et le bord d'entraînement (18.2, 19.2) conçu comme un plateau de moyen de traction, dans lequel l'orientation des axes d'entraînement (D) par rapport à l'axe de rotation commun (A) des deux roues d'entraînement (18, 19) peut être sélectionnée au choix.

13. Appareil de direction (13) selon la revendication 8,
**caractérisé en ce que**
le mécanisme d'engrenage a une poulie à courroie (36) et un moyen de traction (27), et les roues d'entraînement (18, 19) sont conçues comme des roues doubles (18, 19), dans lequel le bord d'entraînement (18.2, 19.2) est conçue comme une poulie à courroie, et dans lequel le moyen de traction (27) fournit une liaison opérationnelle entre la poulie à courroie (36), qui est disposée sur l'arbre d'entraînement (17.1), et le bord d'entraînement (18.2, 19.2) conçu comme un plateau de moyen de traction, dans lequel les axes d'entraînement (D) s'étendent parallèles à l'axe de rotation commun (A) des deux roues d'entraînement (18, 19).

14. Instrument chirurgical (1) ayant un arbre (2), une unité d'actionnement (4) disposée à l'extrémité proximale (3) de l'arbre (2) et un outil (7) disposé à l'extrémité distale (5) de l'arbre (2) avec une pointe d'outil (6) qui peut être inclinée au moyen d'un mécanisme de flexion distale (9) et qui peut être contrôlée par un plateau oscillant (14) qui peut être aligné dans l'espace au moyen de deux entraînements, **caractérisé en ce que**
l'instrument chirurgical (1) a un appareil de direction (13) selon au moins l'une des revendications 1 à 13 pour l'orientation spatiale du plateau oscillant (14).

15. Instrument chirurgical (1) selon la revendication 14,
**caractérisé en ce que**
le plateau oscillant (14) est accouplé de manière cardanique à un arbre principal (21) s'étendent coaxial à l'axe longitudinal (B) de l'arbre (2), et/ou
un élément d'actionnement (8) est monté de manière à pouvoir être déplacé axialement dans l'arbre (2) et est relié de manière opérationnelle à l'unité d'actionnement (4) sur le côté proximal, et/ou le mécanisme de flexion distal (9) de la pointe d'outil pliable (6) constitue des éléments pivotants (11) qui sont disposés à l'extrémité distale (5) de l'arbre (2) et qui sont reliés à l'appareil de direction (13) par des fils de direction (12) s'étendant dans la direction longitudinale de l'arbre (2), et dans lequel une rondelle éventail (22) est de préférence disposée sur l'arbre principal (21) sur le côté distal devant le plateau réglable dans l'espace (14) et augmente la distance radiale des fils de direction (12) depuis l'axe longitudinal (B) de l'arbre (2).
